# EUROPEAN PATENT APPLICATION

(11) **EP 1 785 722 A1**
(43) Date of publication of application: **16.05.2007**
(21) Application number: 05256976.1
(22) Date of filing: 11.11.2005
(51) Int. Cl.: G01N 25/20, G01N 33/48, G01K 17/00, C12Q 1/04, A61B 5/00

(54) **Microbial, viral and mammalian susceptibility to agents that affect cell growth and metabolism, and compatibility of compounds**

(71) Applicant: Stratos Bio Ltd., 1205 Geneva (CH)
(72) Inventor: Kjeldsen, Naja Joslin, 4055 Basel (CH)
(74) Representative: Benson, John Everett

(57) **Abstract**

Method of diagnosing the presence of, or susceptibility to, a condition caused by a pathogenic microorganism in an individual, which method comprises determining whether or not a sample from the individual comprises said microorganism, wherein said determining is performed by placing the sample in conditions in which any said microorganism present in the sample is able to grow, and determining the presence of any said microorganism by detection of the heat generated by said growth using a calorimeter.

## Description

### Field of the Invention

The invention relates to a method of analysing samples for presence of organisms, uncontrolled cell growth and the susceptibility these to various agents and the compatibility of compounds used either alone or in combinations.

Further the invention relates to use of micro-calorimetry to determine heat output of growing and respiring cell systems (10-100 CFU/ml can be identified) and their susceptibility to active agents which can be determined in vivo. The compatibility between active agent (such as antibiotics, antifungals , antivirals and anti carcinogenic agents) used both alone and in combination can be determined within 1 hour to a variability of less than 5% (which is a measure of high reproducibility) as well as the relationship between cell growth, metabolism and anti-microorganism agents, thus to determine new treatment regimens using agents in the most effective concentrations, and without adverse effects.

### Background of the Invention

There exists a need to develop more effective diagnostic and therapeutic methods.

### Summary of the Invention

Microcalorimetric studies of both growing and respiring bacteria and antibiotic action of those have elucidated the mechanism of:
1. Bacterial resistance
2. Emergence of Cancerous Cells
3. AIDS
4. Alzheimer's Disease

Amongst other broad spectrum antibiotics Neomycin and Streptomycin are reported to cause misreading of RNA synthesis. The microcalorimetric results indicate increased protein synthesis of Neomycin treated bacteria followed by increased growth at low concentration of Neomycin. This indicates that the increase in RNA or DNA synthesis depending on whether messenger, ribosomal or transfer RNA is produced acts as a substrate for cell growth (both bacterial and eukaryotic) and viral expression. This increased bacterial growth so called bacterial resistance is an increase due to more nutrients being available to the cells and not a mutation. Increased DNA production and viral expression is seen as a side effect of flagyl metronidazole, commonly used in soft tissue infections and dentistry leading to shingles after treatment, and growth promoting effects in eukaryotes leading to cancer (supported by the NIH JAMA cancer study).

The mechanism of antibiotic or drug induced stimulation of RNA synthesis is as follows:
1. Bacterial resistance Drug induced increased r-RNA synthesis leading to intracellular proteins being produced as nutrients for the bacteria.
2. Cancer: Over treatment by antibiotics leading to increased expression of eukaryotic protein synthesis and thus cancerous growth which is substrate dependent.
3. Increased m RNA synthesis leading to increased DNA synthesis and expression of latent viruses or DNA strands.
4. All of the three above mechanism together in AIDS.
5. Streptomycin induced misreading of RNA synthesis leading to misfolding of

B-pleated sheet in Alzheimers Disease.

### Evidence:

1. Increased respiration and growth of neomycin treated bacteria ( Bacillus pumilus and E. coli) visualised by microcalorimetry (a very sensitive technigue that measures heat-output of both respiring and growing cells).
2. The use of antibiotics as growth promoting agents in agriculture and a US National Cancer Institute study that has shown the link between breast cancer and antibiotic usage The authors of this JAMA study found that women who took antibiotics for more than 500 days - or had more than 25 prescriptions - over an average period of 17 years had more than twice the risk of breast cancer as women who had not taken any antibiotics.
3. The use of antibiotics such as flagyl metronidazole where over use leads to Shingles or expression of latent DNA left over from Chicken pox.
4. Duisenberg claimed as early as 1984 that Aids was not a disease but a break down of the immune system by a massive infection. The timing for the emergence of Aids, gay practises ie anal intercourse, drug taking etc and also the observance of increased incidence of tuberculosis which is transmitted by droplet-infection and/or saliva supports this and could also explain why only some people gets active Aids. According to the Global Fund the emerging middle classes of the development countries are the ones contracting Aids and the incidence of the disease is not as high as previously thought. The Foot and Mouth virus of cattle could fall into the same category as AIDS when kept very dirty conditions the cattle contract massive infections of their feet and mouths.
5. An Animal case study: The Examples of the present application describe the effect of antibiotic treatment on a cat.
Over prescription of Broad spectrum antibiotics such as streptomycin over decades for non serious infections and the increase in incidence of Alzheimer's disease of an ageing population.

Many aspects of the in vivo effects of active agents (such as anti-microorganism compounds) have been insufficiently investigated to date and micro-calorimetry gives us the tools to detect
1. Infection of cells fast and effectively
2. Cell susceptibility to a variety of agents in vivo
3. Effectiveness of treatment in parallel with the treatment (eg in dentistry, veterinary practices, GP's offices and hospital wards)
4. Emergence of adverse effects such as increase in RNA/DNA synthesis or incompatibility of mixtures of agents or uncontrolled growth of cells

The invention concerns use of micro/nano calorimetry to measure the growth/metabolism of cells (bacterial, viral, mammalian) and interactions of anti-microorganism compounds, including:
- measuring the level of infection of a sample (bacteria, virus, mammalian)
- measuring the interactions between anti-microorganism compounds to determine the compatibility of different compounds when used to together,
- determining the interactions of anti-microorganism compounds with 'resistant' microorganisms or their susceptibility to RNA synthesis inhibiting agents,
- determining the effect of anti-microorganism compounds on growth/metabolism of cells or microorganisms, and
- determining the effects of compound structure on activity and mode of action.

The invention also concerns assessment of pathogenic microorganisms in samples from patients using a method that offers many advantages over conventional techniques. A micro/nano calorimeter is able to measure microorganism growth or host cell growth quantitatively, accurately and quickly. The technique also provides other advantages such as the fact that there is no need to pre-treat the sample before analysis, and it is amenable to automation and miniaturization.

Accordingly the invention provides a method of diagnosing the presence of, or susceptibility to, a condition caused by a pathogenic microorganism in an individual, which method comprises determining whether or not a sample from the individual comprises said microorganism, wherein said determining is performed by placing the sample in conditions in which any said microorganism present in the sample is able to grow, and determining the presence of any said microorganism by detection of the heat generated by said growth using a calorimeter.

The invention also provides a method of determining a suitable administration regimen for a patient comprising taking a sample from a patient on a first administration regimen and detecting the effectiveness of the first administration regimen by measurement of the immune response, microorganism load or numbers of malignant cells in a sample from a patient on the first administration regimen, wherein measurement is performed by using calorimetry, and then optionally if the first administration regimen is found to be of low effectiveness selecting a second administration regimen for the patient.

The invention further provides a method of determining whether an anti-microorganism or anti-infective compound is able to cause growth of the microorganism/virus/mammalian cell comprising adding the compound to the microorganism, optionally at a pre-determined low concentration threshold, such as < 10xMIC, <8xMIC, < 2xMIC or < 1xMIC, to determine whether or not growth or heat output of the microorganism/virus/mammalian cell occurs in response to the addition of the compound, wherein growth of the microorganism is optionally measured by detecting heat output from the microorganism/virus/mammalian cell using a calorimeter, and wherein the compound may be alone or in a combination with 1, 2 or more other anti-microorganism or anti-infective agents.

### Detailed description of the invention

Herein the term "microorganism" is understood to include viruses unless the context requires otherwise. Embodiments and features which concern the 'microorganism' aspects are also applicable to the mammalian cell aspects, unless the context requires otherwise.

In one aspect the invention relates to detecting whether infection has occurred, whether stimulation, misreading or inhibition of RNA/DNA synthesis is occurring, the effect of various agents on the infection and the interaction between two or more anti-microorganism compounds, i.e. whether or not they are compatible for use together in vivo. Thus a method of the invention may comprise determining whether said compounds are compatible or incompatible with each other based on whether their activity towards a microorganism increase or decreases when used together (compared to when used separately). The relevant activity is generally causing death or reduction in growth/metabolism of the microorganism or a reduction in uncontrolled RNA/DNA synthesis.

One or more of the compounds which is investigated may act at the cell wall or membrane, or at an intracellular location in the microorganism, such as at a ribosome, or directly on DNA synthesis. In the case where the microorganism is a virus one or more of the compounds may insert themselves into the virus. The mode of action of one or more of the compounds may not be (previously) known. The compounds which are analysed may or may not be related for example to the extent defined herein in the section on related compounds. At least 2, such as at least 3, 4, 5 or more compounds are analysed in the method (by adding them to the same microorganism sample). The compounds may also be tested separately and in any possible combination. In the work described in the Examples of the present application it was found that equimolar amounts gave better biological effects. In one embodiment the compounds used in the method are not related, and thus typically in the method at least two of the compounds will fulfill one or more of the following criteria when compared:
- They will differ in atomic mass by at least 20, such as at least 30 or at least 40,
- One compound will comprise a ring not present in the other compound,
- One compound will comprise a chain of atoms of a length of at least 5 atoms which is not possessed by the other compound.

The compound may be a biological agent, such as a protein or monoclonal or polyclonal antibody of any origin. The compound may be a nucleic acid. The compound may be a gene fragment. The compounds (particularly proteins, peptides and antibodies) may differ in respect of their amino acid sequence, the type or extent of glycosylation (for example differing in the type and number of sugars which are attached) or the type of extent of lipid modification.

Generally the method comprises performing more than one analysis with the compounds, wherein during in each analysis the compounds are added to the microorganism sample in different ratios, which would normally be achieved by providing the compounds in different concentrations (i.e. different molarities). Thus for example in one analysis a first compound will be present at 0.1 to 1% of the molarity of a second compound, or at 1 to 10%, 20 to 30%, 30 to 40 %, 40 to 50 %, 50 to 60%, 60 to 70%, 70 to 80%, 90 to 100%, 100 to 150%, 150 to 250%, 250 to 500%, 500 to 1000%, 1000 to 10,000%. If a first compound is present at a molarity which is 100% of the molarity of the second compound then clearly the compounds are present in a ratio of 1:1 (i.e. an equimolar ratio). Preferably at least 3, such as at least 5, 10, 20, 30 or more analyses are performed with different ratios of the compounds. Typically in the method analyses are performed using at least 2, 3, 5, 10 or more of the specific ratios mentioned above.

Compatibility is detected when the activity of the compounds together is causing a decrease in cell growth and metabolism without an effect on RNA/DNA synthesis, and non compatibility is detected when the activity of the compounds together is less than when the agents are used separately or an increase in heat output is observed followed by increased growth of the cells.

In one embodiment the microorganism is pathogenic (for example for any of the species mentioned in the section below on microorganisms, and preferably for humans). In this case the method may be used to determine the most effective ratio (i.e. the ratio giving highest activity) in which to administer the compounds to an individual who has a condition, or is suspected of having a condition, caused by the microorganism. The ratio may have been found to be an equimolar ratio of each of the compounds.

Another aspect of the invention concerns a method of diagnosis of a condition caused by a pathogenic microorganism comprising determining the presence of the microorganism in a sample from an individual. The presence of the microorganism is detected by measuring heat generated by the microorganism as it grows. The heat is detected by using a calorimeter. Any suitable sample from the individual may be used in the method (for example a sample from a tissue or body fluid which is normally infected by the microorganism or a sample that shows cancerous growth).

### Samples

Any suitable sample from the individual may be used in the method (for example a sample from a tissue or body fluid which is normally infected by the microorganism or a sample that shows cancerous growth). The sample is typically urine, stools, whole blood, plasma, a body secretion (or a swab thereof) or a tissue sample. The sample may or may not be processed before it is analysed in the method of the invention, but in a preferred embodiment it is not processed before analysis. Such processing typically results in a removal of a component from the sample or may result in dilution of the sample (such as dilution of a swab sample). As discussed below substances such as nutrients may be added to the sample to aid growth of the microorganism. Such additions of substances are not understood herein as being "processing" of the sample.

Typically the sample (at the point of being taken from the individual) has a volume of less than 10 millilitres, such as less than 5, less than 3 or less than 3 millilitres, down to for example 25 ul or less. The sample which is analysed by calorimetry typically has a volume of less than 10 millilitres, such as less than 5, less than 3 or less than 3 millilitres, down to for example 25 ul or less.

### Individuals

The individual on whom diagnosis is performed may be from any species, and is typically a bird or mammal. The individual is preferably human or an animal. The individual may be one which is in a state that renders it susceptible to infection by the microorganism, or susceptible to cancer.

### Microorganisms and cells which are used and/or detected in the methods of the invention

Any suitable microorganism may be used or detected in the methods of the invention. The microorganism is typically a bacterium, yeast, fungus or protozoa or virus. A tumor or malignant cell culture may be used or detected in the method of the invention.

### Conditions under which detection is performed

In the method of measuring compatibility the relevant compound(s) are simply contacted with the microorganism or cell and the heat generated is detected. No further components need to be added to sample, and thus for example the microorganism or cell does not need to be in conditions in which growth will occur (as respiration studies can also be used to detect susceptibility to a compound depending on the mode of action of the compound).

Typically detection of heat is carried out for at least 1 hour to 48 hours, such as 3 to 24 or 8 to 12 or 4 to 5 hours after addition of the compound(s). Preferably continuous measurement of heat detection is performed, such as at least once every 10 minutes, such as over any of the above specified time periods. For example over a 24 or 48 hour period cell death can be demonstrated (and thus the bacteriocidal effect of antibiotic in vivo), or over a 2 hour period inhibition of growth (and return to baseline) or compatibility of compounds.

In one example the sample is placed in conditions which studies the metabolism of the cells and effects of various agents on the same. In another example the sample (for example from an individual) is placed in conditions which allow growth of the microorganism or cell to be detected. Thus typically nutrients are added to the sample which aid growth of the microorganism or cell. The medium may be a defined medium, blood broth, nutrient broth, a defined medium for a known microorganism, cell culture medium or a saline solution (for respiratory studies).

The sample may be placed in a temperature which allows growth/heat output of the cell or microorganism to be detected, such as in a temperature of 20 to 45 degrees C, typically in a temperature of 30 to 40 degrees C, such as at a temperature of 35 to 38 degrees C or at 37 degrees C.

The sample is typically placed in conditions allowing growth of the cell or microorganism for 3 to 12 hours, and typically for less than 8 hours, such as less than 6 or less than 4 hours.

As mentioned above in the method heat produced by growth of the microorganisms is detected. In one embodiment the heat produced is analysed over time, for example in the form of a graph of heat produced versus time (or power versus time).

The micro/nano calorimeter which is used in the method of the invention is normally one which is capable of continuously measuring heat effects (enthalpy changes) of a sample. In one embodiment the calorimeter is capable of detecting the heat output of 10² to 10⁵ CFU/ml of microorganisms, such as of any of the species mentioned herein. Typically the calorimeter is capable of detecting a rate of heat change of 1uW or 1nW. The calorimeter may be capable of measuring heat output simultaneously from more than one sample, such as from at least 4, at least 20, at least 50 or at least 100 samples. During analysis of the heat output the sample may be in aerobic or anaerobic conditions.

The invention also provides a kit for carrying out a method according to any of the preceding claims comprising a calorimeter suitable for use in said method. The calorimeter may be specifically adapted so that any of the types of samples mentioned herein can be analysed and/or data concerning heat output from a sample can be provided to a computer or database for analysis of the data.

### Detecting side effects or detrimental effects of anti-microorganism compounds

One aspect of the invention is based on the effect of low concentrations of anti-microorganism compounds on the growth of cells. Such cells can be bacterial cells (such as an any type or species of microorganism mentioned herein), preferably bacterial cells, or eukaryotic cells. It has been found that certain anti-microoorganism compounds, especially compounds that have an effect on RNA synthesis, can cause cells to grow and thus may explain bacterial resistance (increased intracellular protein synthesis providing nutrients for the bacteria in vivo), viral infections (increased synthesis of mRNA and the expression of dormant DNA fragments, e.g. the appearance of shingles after administration of antibiotics) and also the emergence of HIV (a mixture of the two above mechanisms eventually rendering the immune system ineffective). The same effect in eukaryotic cells would give rise to tumour growth. Thus previously observed apparent resistance to anti-microorganism compounds may be due to stimulation of growth of the relevant microorganism at a concentration of anti-microorganism compound which is too low or alternatively due to stimulation of eukaryotic growth when administered in large excess or when no microorganism is present.

The effect of the anti-microorganism compound on cells is consistent with the known growth promoting effects of antibiotics. This also suggests a mechanism for anti-microorganism compounds to cause cancer.

The present work thus provides evidence of actions of anti-microorganism compounds which can cause detrimental effects in the individual being treated. Such actions will contribute for example to some of the side effects observed when using anti-infective compounds.

Accordingly the invention provides a method of determining whether an anti-microorganism compound (e.g. an anti-infective compound) is able to cause increased growth or respiration of the microorganism/virus/cell comprising adding the compound to either a respiring or growing microorganism, optionally at a pre-determined low concentration threshold, such as < 10 X MIC, < 8 X MIC, < 2 X MIC, or < 1 X MIC. The threshold may be less than 0.01ug/ml, 0.01 ug/ml to 10ug/ml, such as 0.1 ug/ml to 1ug/ml. A single anti-microorganism compound may be added or 2, 3, 4 or more anti-microorganism compounds may be added.

For neomycin (with a known mode of action of cause misreading during RNA synthesis) an increased heat output can be observed at concentrations of < 1ug/ml or in combinations with other antibiotics. For respiring E.coli all concentrations of Neomycin caused an increased heat output.

This can be used to determine whether or not growth of a microorganism/virus or cell occurs in response to the addition of the compound. Preferably growth of the microorganism/virus or cell is measured by detecting heat output from the microorganism/virus or cell using a calorimeter, such as in any of the ways discussed previously. The microorganism is generally any microorganism that causes disease, for example any such microorganism disclosed herein.

If the said compound is found to cause increased growth of microorganisms at the pre-determined low concentration, then appropriate action can be taken in the manner in which it is used, for example it can be administered to an individual suffering from a condition caused by the microorganism or at risk of such a condition, at a high dosage which would ensure killing of the microorganism. It can also be used to monitor infections in patients (both animals and humans) and thus administer anti-infective agents when necessary and stop treatment when the microorganism/virus or cell is dead (indicated by return to baseline of heat output, confirmed by viable count measurements).

In the case of neonates or children the identified risks of anti-microorganism compounds means that administration of such compounds should be reduced. Thus the invention provides a method of administering an anti-microorganism or anti-infective compound to a neonate or child individual suffering from, or at risk from, a disease condition according to an improved schedule which reduces detrimental effects, wherein in the improved schedule one or more dosages are decreased by at least 1.5 fold in comparison to an existing schedule, wherein the individual is optionally below the age of 10 or below the age of 5 or 1 years old. The reduction may be 1.5 fold to 100 fold, such as 2 fold to 50 fold or 10 fold to 20 fold. The reduction may be 1 to 250 mg/kg, such as 5 to 100 mg/kg or 10 to 50 mg/kg.

Further the invention provides a method of determining possible detrimental effects of an anti-microorganism compound on a eukaryotic individual comprising determining whether said compound:
- causes increased growth of the eukaryote or cells from the eukaryote,
- causes cells of the eukaryote to become cancerous, or
- causes increased production of various types of RNA or protein in cells of the eukaryote,
wherein said determining is optionally performed using a calorimeter.

The compound may have been administered to the individual for the purpose of treating a condition caused by a microorganism against which the compound acts. The individual may be any of the types of, or species of, individual mentioned herein, and is preferably human.

The above methods may further comprise elucidating the mechanism of the effect of the compound on a cell, such as investigation of RNA or DNA synthesis and/or protein synthesis levels of the cell.

The invention further provides a method of selecting an anti-microorganism compound to administer to an individual suffering from a condition caused by the microorganism or at risk of such a condition, comprising testing whether a candidate anti-microorganism compound is able to cause increased growth of the microorganism or inhibit growth or kill the microorganism at relevant concentrations or in combinations with other anti- infective agents (wherein optionally the compound is then administered to the individual). Typically a compound is selected which:
- kills the microorganisms or stops growth and does not (for example at any concentrations) cause increased heat output by growth or respiration indicating stimulation of RNA synthesis
- only causes growth below a pre-determined low concentration of compound.

Mixtures of agents to be administered to patients can also be tested in the same way (where such mixtures include medications for different conditions). The invention teaches that special care should be applied when administering these agents to children, neonates or animals as excessive doses can cause inducement of RNA synthesis in eukaryotic cells.

Given that low concentrations of anti-microorganism compounds have been shown to cause the above described detrimental effects, clearly it is advantageous to adopt administration schedules in which higher dosages of the compound are given but exert caution in over administration and prolonged treatment. Thus the invention provides a method of administering an anti-microorganism compound to an individual suffering from, or at risk from, a disease condition according to an improved schedule which reduces deleterious effects, wherein in the improved schedule one or more dosages are increased by at least 1.5 fold in comparison to an existing schedule at short time intervals. The increase may be 1.5 fold to 100 fold, such as 2 fold to 50 fold or 10 fold to 20 fold. The increase may be 1 to 250 mg/kg, such as 5 to 100 mg/kg or 10 to 50 mg/kg. The individual to whom increased doses are administered is preferably not a child, but is typically at least 15 years, such as at least 20 or 25 years old.

Typically 1, 2, 3, 4, 5 or more of the administrations in the schedule may be increased 1.5, 2, 2.5, 3, 4 or more fold (for example in a 2 fold increase the amount (ug) of compound which is administered would be doubled).

The anti-microorganism/anti-infective compound in question may be any of the compounds mentioned herein, and is preferably an antibiotic, such as a broad spectrum antibiotic and/or an antibiotic which acts at the ribosome of the microorganism/eukaryote.

### Anti-microorganism compounds

The anti-microorganism compound may be Ampicillin, Chloramphenicol, Erythromycin, Nalidixic acid, Tetracycline, Aminoglycoside (such as streptomycin, kanamycin, gentamicin, tobramycin, amikacin, netilmicin and neomycin), tetracycline, minocycline and doxycycline, Spectinomycin, lincomycin, clindamycin, a macrolide (such as Erythromycin, clarithromycin, azithromycin or spiramycin), Fusidic acid, Rifampin, rifamycin, rifampicin, Quinolone (such as nalidixic acid, ciprofloxacin, oxolinic acid, Ofloxacin, norfloxacin, levofloxacin, lomefloxacin, sparfloxacin), sulfonamide, sulfone, Trimethoprim, methotrexate, pyrimethamine, Para-aminosalicylic acid (PSA), Dapsone, Isoniazid (INH), Spectinomycin, a Penicillin, flagyl metronidazole, Actinomycin, a sulphadrug, vancomycin, adrenamycin, daqucomycin, rifamycin, heparin, afo/3, streptoygidin, or Streptovaricin.

### Administration of agents

As mentioned above the invention includes administration of particular agents. Such an agent will generally need to be formulated into an appropriate composition to make it suitable for administration. The agent will typically be administered to an individual (human or animal) in need thereof.

The formulation will depend upon factors such as the nature of the substance and the individual to be treated. Any such substance may be administered in a variety of dosage forms. It may be administered orally, parenterally, subcutaneously, intravenously, intramuscularly, intrasternally, transdermally or by infusion techniques. The substance may also be administered as suppositories.

Typically the substance is formulated for use with a pharmaceutically acceptable carrier or diluent. The pharmaceutical carrier or diluent may be, for example, an isotonic solution.

An effective amount of substance is administered. The dose may be determined according to various parameters, especially according to the substance used; the age, weight and condition of the individual to be treated; the route of administration; and the required regimen. A typical daily dose is from about 0.01 to 500 mg per kg, preferably from about 0.1mg/kg to 10mg/kg of body weight.

The following Examples illustrate the invention:

### Examples

A micro/nano calorimeter is an apparatus that can measure the heat lost or gained during all chemical, physical and biological processes. The heat produced during these processes creates a temperature difference between the sample in the test chamber and its surroundings. The intensity of the process can thus be established through continuous monitoring of the temperature difference. The instrument used in the work is a heat conduction microcalorimeter and is designed to minimise temperature difference and encourage the most rapid heat flow away from the reaction site to a large heat sink (ΔT<10⁻⁴ °C).

Micro/nano calorimetry is a non-destructive and non-invasive direct technique that does not require any prior sample treatment (like the lysis-filtration with impedance detection method). The analysis is also not limited to a physical state of a sample i.e. the calorimeter can deal with any sample form: solids, liquids, gases, transparent and non-transparent samples alike can be analysed. The colour of the sample is also not an issue using calorimetry. The calorimeter's indifference to colour and substrate nature makes it an ideal system for analysing the red, complex, suspended, cell composition of blood. Calorimetry provides a favourable advantage to the classical method of blood culturing as it can analyse very small samples of blood.

If an uninfected sample is placed in a calorimeter, one would expect there to be a clinically insignificant number of organisms present per ml (i.e. <10⁴ organisms/ml). It would thus take some time for the organisms in the sample to grow (to >10⁴ organisms/ml) and produce enough heat so that it can be detected in the calorimeter. Conversely, an infected sample will have >10⁴ organisms/ml. When this sample is placed in a calorimeter, it would have enough organisms to generate an immediate detectable level of heat.

### Media Dependency

The media employed in classical microbiological assays are almost invariably complex, containing such constituents as beef extract, yeast extract, blood components and peptone. Such media may not be the optimal choice for a microbiological assay.

Constituents of complex media, such as yeast extract and peptone, will vary from batch to batch; even different samples of a complex medium, derived from the same batch of its constituents, may be different after autoclaving. The response of a culture to small changes in medium composition could be a serious limitation to any microcalorimetry assay; hence, a complex medium may not be the optimal choice.

To obtain defined, reproducible media, synthetic media of known chemical composition are better.

The pH of the medium should be constant throughout incubation. Fluctuation of pH will have the following effects: (i) metabolism may vary as a function of pH, (ii) the heat associated with the removal of substrates and the appearance of metabolites will depend upon their respective degrees of protonation, and (iii) the activity of candidate substance which is being tested may vary as a function of pH.

Antibiotics are known to combine with several medium components. For example, binding to proteins and salts is common and due to these effects, the active concentration of the antibiotic is often less than calculated. In addition components of the media may affect the death rate or rescue treated cells and/or inhibit their interaction with the antibiotic. Using a defined medium, the effect of medium components on antibiotic action can be investigated and controlled.

In addition using defined media for susceptibility studies have elucidated and quantified the growth promoting effect of certain antibiotics (demonstrated both with Neomycin and Bacitracin) and can be used to investigate agents for medical use.

### Importance of a Reproducible Inoculum

Liquid nitrogen refrigeration (-196°C) may be used in the preservation of microorganisms. Inocula stored at this temperature are superior to those stored at higher temperatures in that they retain constant viability over long period.

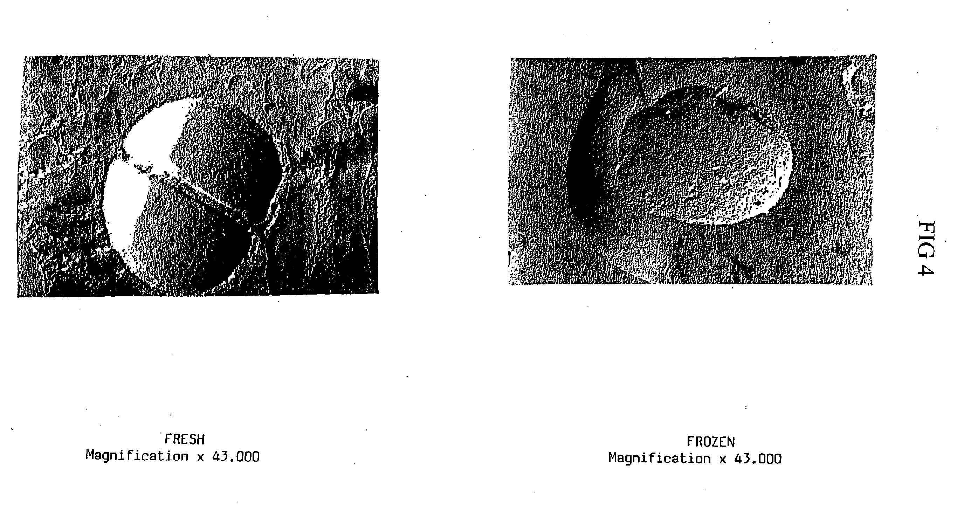

In addition to long time viability inocula recovered from storage in liquid nitrogen give standard inhibition zones in plate assays and standard turbidity for a standard dose of antibiotic. More importantly consistent electron micrographs showed no damage of the cell wall or membrane on freezing and thawing (Figures 4,7and 8). Consistent electron micrographs also showed the anticipated effects of antibiotics on cells given their mode of action.

### Importance of Oxygen Tension

Oxygen tension is a crucial factor in the growth of facultative anaerobes and aerobes. The oxygen available for microbial growth is dependent on oxygen solution rate and this is dependent on fermentor conditions e.g. size, volume of medium, flushing rate, stirring rate and number of baffles. Since the oxygen tension is influenced by so many factors which all have to be accurately reproduced for reproducible assays it is better to adopt strict aerobic or anaerobic conditions.

### Organisms

All bacterial cell preparations were suspended in Ringers solution or 10% DMSO and frozen and stored under liquid nitrogen. The viable count of thawed inocula was determined using the surface spread plate method.

### The Calorimetric Medium

a) For B. pumilus a simple glucose/salts medium was employed with a glucose concentration of 0.1 % w/v.
b) For Bord. bronch. The above salts medium was used with the addition of nicotinic acid, methionine and glutamic acid. Glutamic acid was used at a concentration of 0.2% w/v.
c) For M. flavus a semidefined medium was employed, using the same salts as a B. pumilus but also Na-citrate, biotin and protease peptone. In the calorimeter a concentration of 0.4% protease peptone was used. All medium constituents (except protease) were analytical reagents. The calorimetric medium was therefore a final concentration of 100ml of either solution a, b or c freshly autoclaved for 15 mins exactly at 121°C, 151b/sq inch. Glucose was added to the appropriate solution after autoclaving.

### The Calorimetric Incubation Vessel

The bacterial cultures were grown in a fermenter vessel of approximately 200ml capacity which had a flat ground-glass rim. To the vertical face of the vessel close to its base, equidistantly spaced around the circumference, ports were fitted for an oxygen electrode and for sampling/inoculation. The fermenter was closed with a flat flanged glass lid which had five ports accommodating respectively, gas inlet tube, gas exhaust (via air condenser), inlet and outlet flow-lines to and from the calorimeter. The fermenter and lid was sealed with a p.t.f.e. gasket and clamped with a metal clip. The fermenter was placed on a magnetic stirring unit in a water jacket which allowed circulation of the thermostatically controlled water at 30°C or 37°C. The fermenter which contained a Teflon coated magnetic stirring bar, was sterilised by autoclaving.

### The Calorimetric Incubation

For a typical calorimetric incubation the medium was passed through the calorimetric vessel at the same flow rate and temperature as those used in the experiment to establish a steady baseline deflection. The following operations then took place to a strict timetable. The liquid nitrogen stored ampoule was thawed at 38°C for 3 min and then stirred for 20 sec on a whirlimixer. The bacterial suspension (0.5-1.5ml) volumes was inoculated into the medium (100ml) two minutes after completion of thawing. A Gilson automatic pipette was used for inoculation. The calorimeter inlet flow-line was placed in the incubation medium 30 seconds after inoculation. The flowing incubation medium reached the calorimetric vessel two min after inlet flow-line insertion. At this time the recording of the p-t curves was started. Eight min after inoculation the outflow from the calorimeter was returned to the fermenter (after the "baseline" medium had run to waste but before the inoculated medium ran to waste) thus making a closed system. Aeration was started as soon as the inlet tube was inserted in the fermenter. Microbiological purity of the culture was checked by plating out a sample of the culture at the end of each incubation on nutrient agar (lab M).

The flow-microcalorimeter cannot be sterilised by conventional methods but can be decontaminated by successively pumping through solutions of 0.1% w/v NaOH and finally sterilised deionised water. (For M. Flavus it was necessary also to use 70% w/v ethanol as well to remove any traces of peptone in the flow lines).

### Presentation of Calorimetric Results

The output of calorimetric experiments is a plot of power (energy evolution per unit of time) as a function of time. The unit of power (p) is the Watt (W) and the unit of time is the hour (h). In some experiments seconds (s) were used as time unit.

### Analyses of P-T Curves

The p-t curves obtained by growing Bord. bronch. in various concentrations of glutamic acid all have the same general appearance. A small peak after 6h 30min and a broad peak after approximately ten hours. The small peak suggests some regular change in metabolism e.g. change in oxygen tension, in toxin production or in C or N source. This change in power, was quantitative and reproducible. The peak height increased with increased glutamic acid concentration although at an initial concentration of 0.2% glutamic acid no change could be detected in glutamic acid concentration after 7h. The peak height varied from 20-40% of full scale deflection when increasing glutamic acid concentration from 0.1% to 0.2% and again to 80% of full scale deflection when using 0.3% glutamic acid. This was also true for the broad peak although the power increase from 0.2-0.3% glutamic acid was only 40%.

Very little variation in the slope of the small peak at different concentrations of glutamic acid could be observed - suggesting that the kinetics of metabolism were the same for different concentrations of glutamic acid.

To be able to distinguish the small peak at all is a great improvement in the sensitivity of the calorimeters and the reproducibility of our assay procedure. Very few workers have achieved a greater sensitivity than that of 100µV full scale deflection which is required to distinguish the small peak for Bord. bronch. Differences in p-t curves of different bacteria can be used as a means of identifying them, but differences in p-t curves were usually media dependent and when media constituents were changed completely different p-t curves arose. These changes were indicative of changes in metabolism which were reproducible and quantative.

### MICROCALORIMETRIC ANALYSIS OF NEOMYCIN SULPHATE USING BACILLUS PUMILUS AS TEST ORGANISM

Neomycin sulphate is a broad spectrum antibiotic and is active against Gram positive and Gram negative organisms.

### Antibiotic Incubation

The MIC for neomycin sulphate against B. pumilus was found to be 0.1 µg/ml with an inoculum of 1x10⁵ cfu/ml and the effect was bacteriostatic. However when the concentration of organisms was 5.1 x 10⁶ cfu/ml which was the inoculum used in the microcalorimetric incubations the MIC was increased to 2 µg/ml; the effect was also bacteriostatic.

When added after a 25% increase power output 0.5 x MIC (1µg/ml) neomycin gave rise to a massive power output 2-4h after addition. The effect of neomycin when added after an increase in power output of 15% of full scale deflection was only marginally larger than that observed for the normal growth curve.

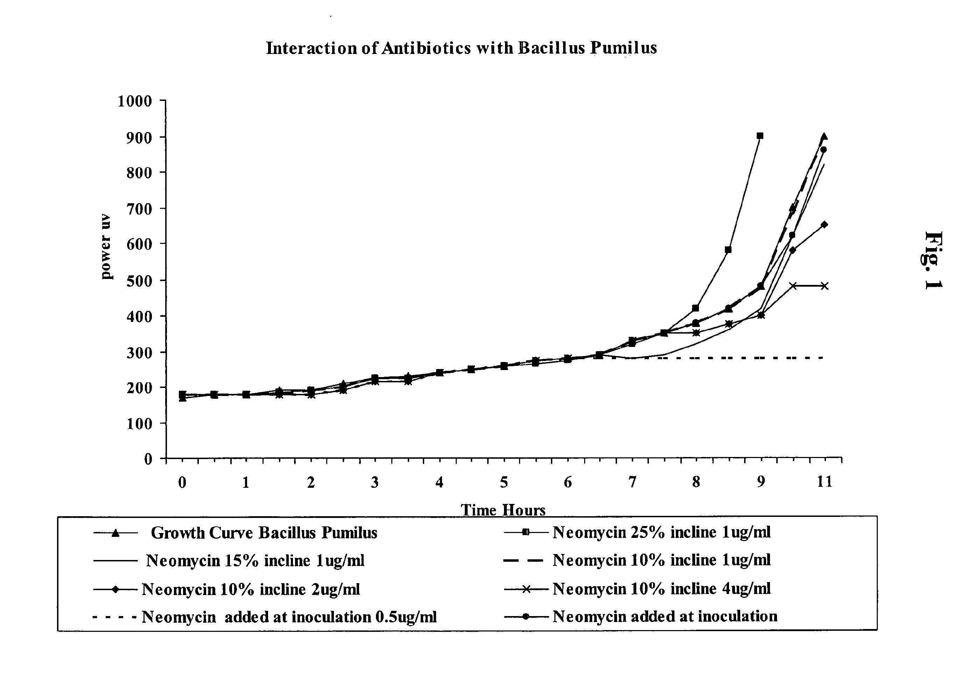

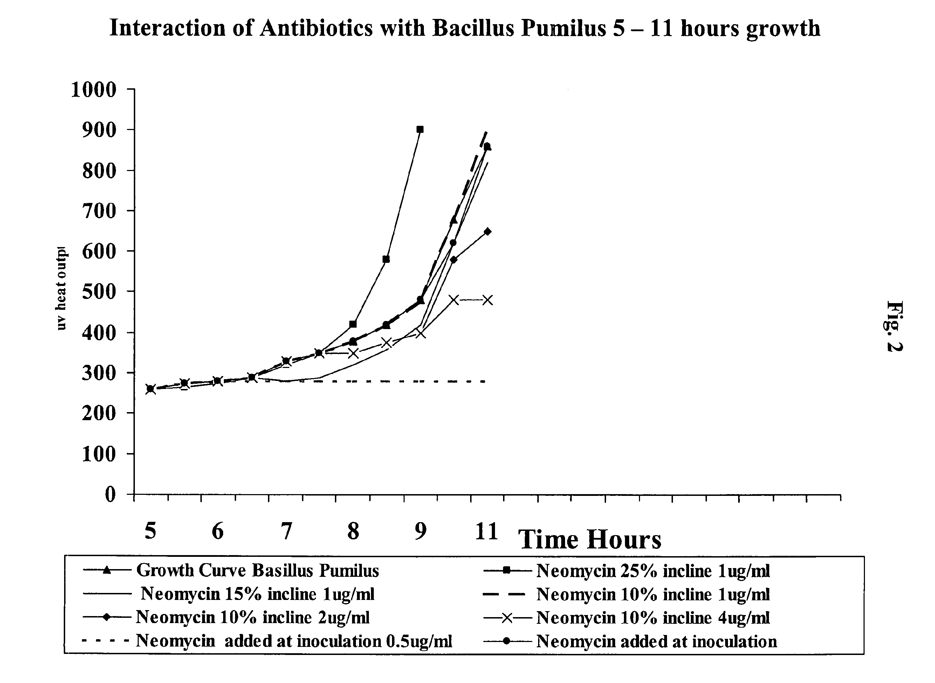

When 0.5 and 1 x MIC (1-2 µg/ml) neomycin was added after an increase of 10% of full scale deflection the same effect was observed (Figs. 1 and 2). When higher concentrations of antibiotic were added (Figs. 1 and 2) a decrease in power output was observed. These observations indicate a mechanism of action for bacterial resistance to antibiotics, i.e. that when an antibiotic is used at suboptimal conditions (e.g at a lower concentration than the MIC) , RNA synthesis is initialised that will within a short period of time provide proteins used as nutrients for the bacteria and thus the bacteria may grow at a rate which is greater than in the absence of antibiotic, and thus give rise to the notion that resistance is observed. This is supported by observations made during other uses of these antibiotics and is consistent with their growth promoting effects on poultry, dairy herds and pigs. More seriously when overtreating patients with antibiotics that have this effect eukaryotic protein synthesis may be initialised and act as a substrate for eukaryotic cell growth, i.e. cancer, or mRNA may be produced leading to expression of previously latent DNA such as observed in shingles infections after treatment with flagyl metronidazole an antibiotic commonly used in dentistry (see the section on neomycin treated E.coli where bacterial respiration is increased immediately on treatment with antibiotic indicating increased RNA synthesis).

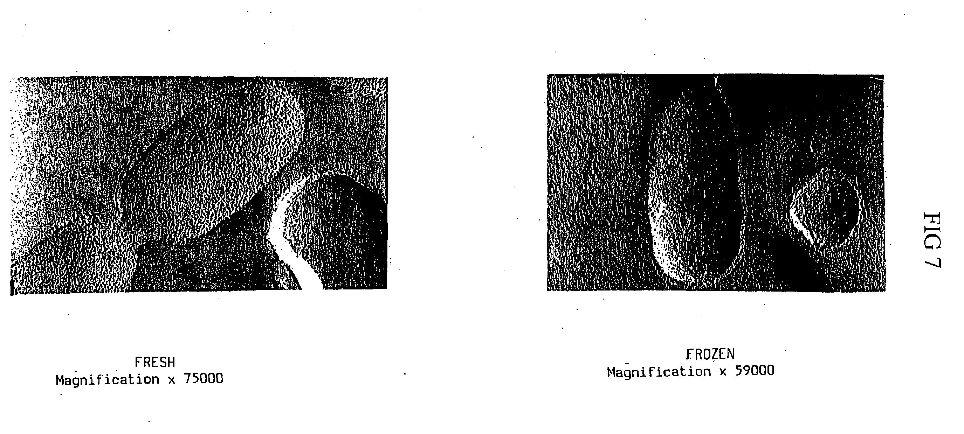

No difference between fresh and frozen bacteria could be observed by EM (Fig 7).

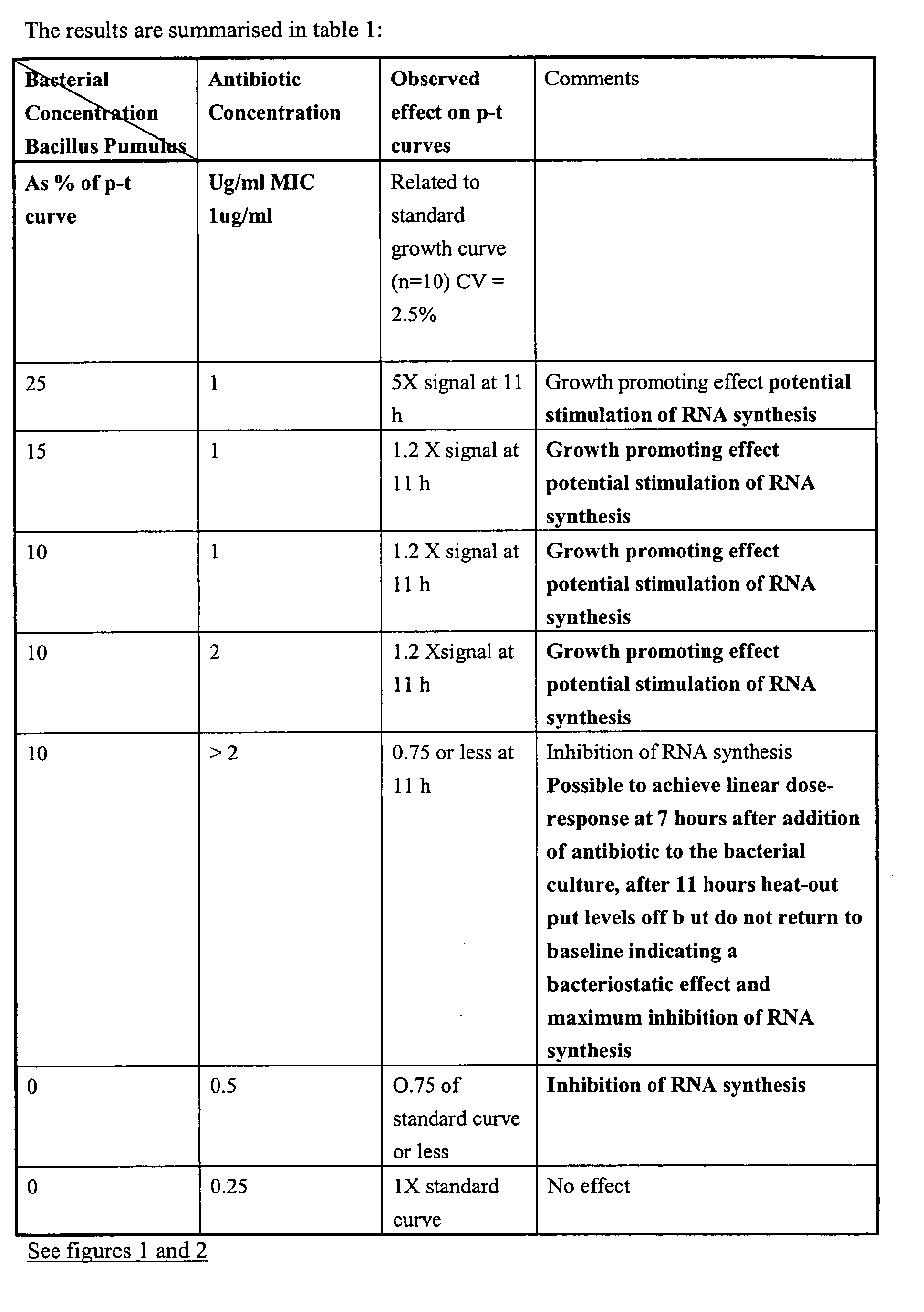

### Assay of Znbacitracin against Microccous Flavus

It was found that addition of the antibiotic half way up the peak maximum would be convenient as the cells were growing exponentially at this point and the increase in power output was due to growth rather than baseline variation. This point occurred at an increase in deflection of 29.5% from the baseline value. See table 2:

| **Bacterial Concentration Microccocus Flavus** | **Antibiotic Concentration** | **Observed effect on p-t curves** | Comments |
|---|---|---|---|
| **As % of p-t curve** | **Ug/ml MIC & MBC 0.2 ug/ml** | Peak at 54% deflection N = 10, CV = 1.5% | |
| 50 | 0.1-1 | Linear log dose response curve 2 hours after addition of antibiotic CV 1.8% | Peak of growth curve occurs 60 min later than standard curve |
| 50 | 1-4 | Linear log dose response curve 20 min after addition of antibiotic cv = 1.4% n= 10 | 4 ug/ml largest dose that could be distinguished any higher dose s gave identical dose response curves |
| 0 | 0.2-0.3 | 1.4 standard curve CV =2.3% , n=10 | Initial growth promoting effect - delayed peak after 9 hours |
| 0 | 0.6-1 | Total inhibition of growth | Bacteriocidal effect confirmed all bacteria are dead |
| 50 | 0.02 | Standard growth curve | No inhibition observed |

### Figure 3

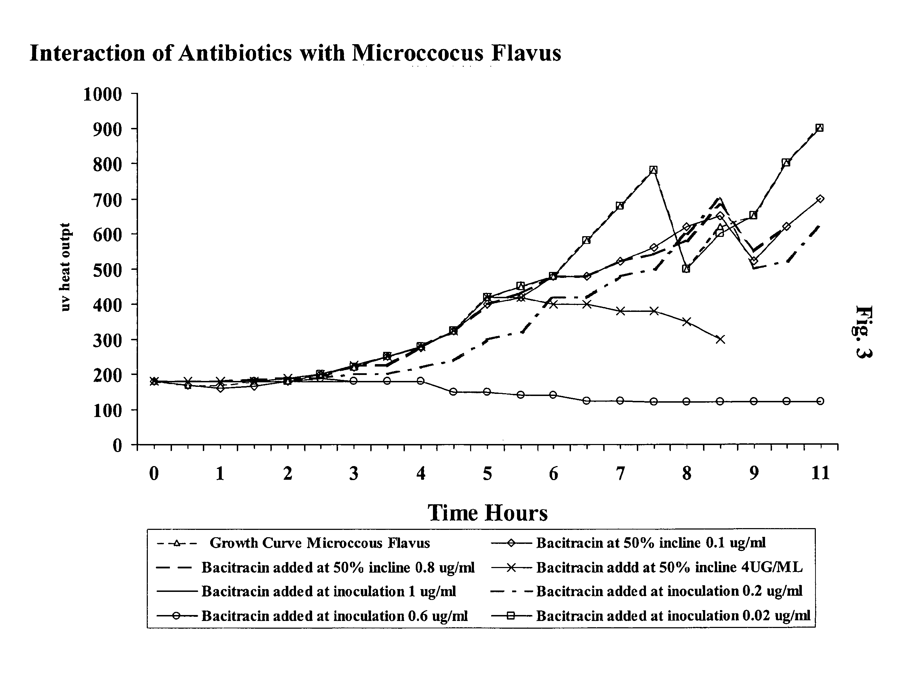

When adding Znbacitracin over the concentration-range (5 x MIC - 20 x MIC) the p-t curves (Fig. 3) showed the same general appearance: they were held at a plateau for a varying amount of time and then decreased towards a baseline value. A very rapid response of M. flavus to ZNB was observed making it possible to develop an assay only 20min after addition of the antibiotic. This was indicative of a possible effect on the bacterial membrane of the bacteria since peptidoglycan synthesis takes place between the cell membrane and the cell wall of the bacteria and inhibition of growth would not give much a rapid response.

When using concentrations of antibiotic from 0.02-0.8 µg/ml (0.1 x MIC-4 x MIC) the general trend was: a power plateau immediately after addition of the antibiotic for varying periods of time and then an increase in power followed by a further small peak which was observed at varying times - but in all cases later than for the control curve.

At concentrations lower than 0.02 µg/ml no variation in p-t curves could be observed and at concentrations higher than 4 µg/ml no further response could be recorded. The initial growth promoting effect at 0.2 -0.3 ug/ml of Bacitrazin when added at inoculum is consistant with observations of resistance patterns of penicillins also cell wall inhibiting agents and could be due to an effect on RNA synthesis similar to neomycin.

### EFFECT OF BACITRACIN ON THE CELL SURFACE OF MICROCOCCUS FLAVUS

No difference could be observed between electron micrographs of M. flavus taken from frozen and fresh samples. (Fig. 4). This suggests that no damage to the bacterial cells occurred during the freezing process.

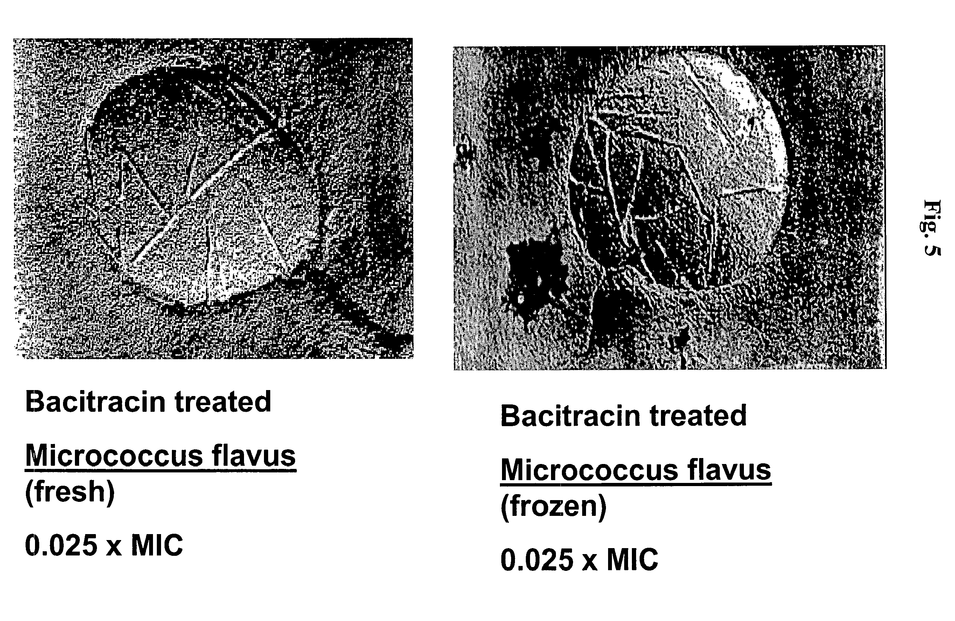

Fig. 5 shows EM's of samples treated with Znbacitracin in the same manner as in the antibiotic assay. Protrusions on the cell wall can be observed even at concentrations of 0.05 µg/ml (0.25 x MIC). No differences could be observed between fresh and frozen cells treated with antibiotic, which is as expected since no observable damage had occurred to the bacterial cells during freezing/thawing.

The sensitivity of this assay is very high as levels of 0.02 µg/ml can be measured. This is because much better control of the different parameters is achieved than would be possible for a plate assay. This is interesting as no ordinary microbiological assay can compete in sensitivity and this is achieved under conditions which more accurately represent *in vivo* conditions than any other assay available.

The reproducibilities of these two assays ±1.4% and ±2.3% are excellent. They fall in the category of reproducibilities of quantitative chemical analysis which is extremely good for a microbiological bioassay. It is remarkable that these reproducibilities can be achieved using only a semi-defined medium and therefore not achieving as great a degree of control over parameters as when using a defined medium

### MICROCALORIMETRIC BIOASSAY OF POLYMYXIN B SULPHATE USING BORDETELLA BRONCHISEPTICA AS TEST ORGANISM

Polymyxin B sulphate is a membrane active antibiotic mainly active against Gram negative organisms. Bord. bronch, is the reference test organism for this antibiotic. The defined medium finally arrived at for this organism has been described previously. Ordinarily Bord. bronch, is grown in complex medium containing proteose and yeast extract.

### Microcalorimetric Analysis of P-T Curves

After successful freezing of Bord. bronch p-t curves for growth in different concentrations of glutamic acid from 0.1%-0.5% were obtained. It was concluded that a medium containing 0.2% glutamic acid would be sufficient for a good deflection in the microcalorimeter minimising the possibility of the bacteria clogging in the microcalorimetric cell or in the teflon tubes leading to and from the microcalorimetric cell. A pH of 7 was used in all the following experiments as this is the optimum pH for polymyxin - lipid binding.

It was of vital importance to maintain a constant oxygen tension; this was achieved by using saturation concentrations of oxygen. It was decided to use the height of the small peak as full scale deflection for the antibiotic assay as the increase in power was exponential up to this point and the peak occurred at a convenient time. This choice of assay point was empirical. The inoculum was 1.1 ± 0.06 x 10⁸ cfu/ml for all the microclalorimetric incubations.

| **Bacterial Concentration Bordetella Bronchiceptica** | **Antibiotic Concentration** | **Observed effect on p-t curves** | Comments |
|---|---|---|---|
| **As % of p-t curve** | **Ug/ml MIC & MBC 2 ug/ml** | Peak at 39% deflection N=10, CV=2.6% | |
| 18 | 0.35 -2 ug/ml | Linear log dose response 10 min after addition of antibiotic CV 2.5% | All curves return to baseline confirmed bacteriocidal action |
| 18 | 0.07 ug/ml | No effect on growth curve | |

### Fig6

Analysis of the p-t curves of 0.2% glutamic acid revealed that the small peak occurred between 5h 30min-6h 30min. The average peak height was 39% of full scale deflection (at 10µV = 157.05µW). The standard deviation in peak height was ± 2.6% over ten measurements.

Analysis of control incubations without antibiotic and with 0.2% glutamic acid showed that after a lag of 1h 20min OD and heat output increased exponentially up to the first peak (39% deflection, 5½-6½h). The viable count during this period increased from 1.1 ± 0.06 x 10⁸ to 3.5 ± 0.18 x 10⁸ cfu/ml, pH did not change. Glutamic acid utilisation during this period was not detectable.

### Antibiotic Incubations

It was found that one doubling of cells (1.9 ± 0.1 x 10⁸ cfu/ml) occurred at an increase in deflection of 17.5% (2 determinations). This was a suitable point for addition of antibiotic as the cells at this point were growing exponentially and the increase in power was great enough to ascertain that any increase in deflection was due to growth rather than a baseline variation.

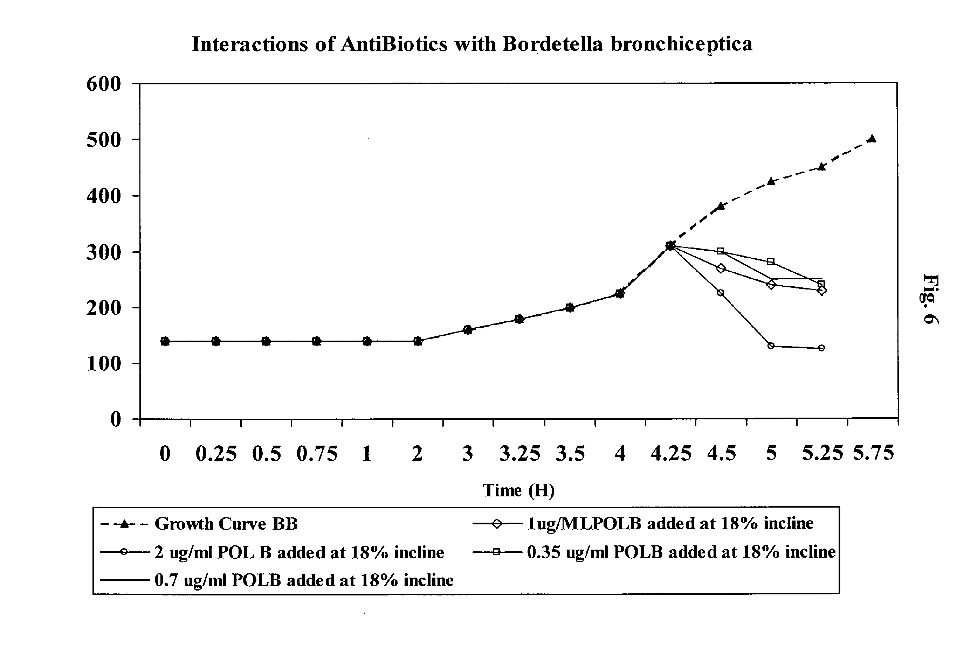

A concentration of 0.5 x MIC (MIC and MBC: 2µg/ml) was added and a p-t curve with a sharp decline in heat output within 10 min of addition resulted. A range of different concentrations of polymyxin B sulphate was assayed in the microcalorimeter Fig. 6. It can be seen that the rate of change in decline of the p-t curve increases with increase in concentration of antibiotic. At concentration larger than 3.5 x MIC the declines were identical. At a concentration of 0.033 x MIC (0.07 µg/ml) no response to the antibiotic was observed. At small concentrations of antibiotic the decline became very shallow.

The sensitivity range for the assay of polymyxin using the slope as response was 0.35µg/ml (0.175 MIC) - 2µg/ml (1 x MIC). Although a response was seen at lower concentrations of antibiotic the decline in power was small and difficulties arose in distinguishing slopes at these concentrations. The standard deviation for this assay was ± 2.9% (10 measurements). It can be concluded that the uptake of polymyxin was very quick (a response could usually be observed within 5 min of addition of the antibiotic) and that the effect was irreversible for most concentrations of antibiotic as the decline in power decreased to a baseline value. This was in accordance with the mode of action of the antibiotic being bactericidal at MIC values.

### Effect of POLB on the Cell Surface of Bordetella Bronchiseptica

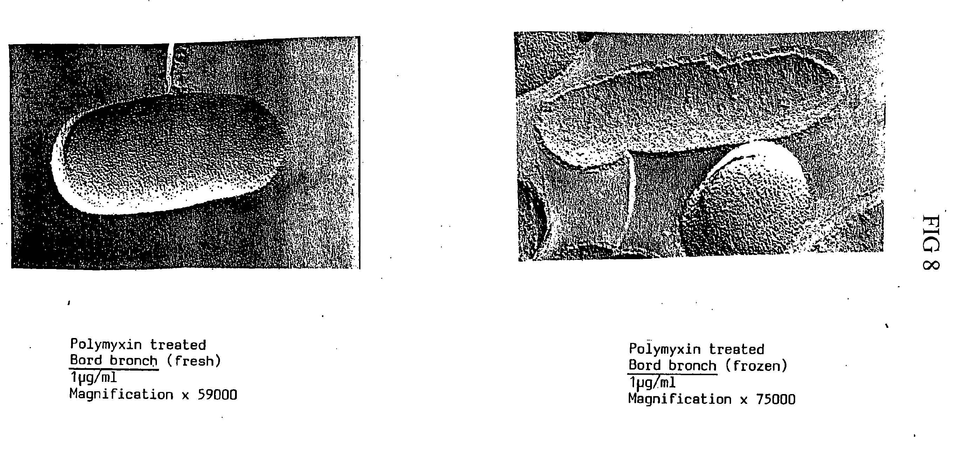

No difference could be observed between electron micrographs of Bord. bronch, taken from fresh and frozen samples (Fig. 7). This suggests that no damage to the bacterial cells occurred the freezing process. After incubating fresh and frozen bacteria with the antibiotic, in the same manner as in the antibiotic assay at concentrations of 1µg/ml (0.5 x MIC) no differences were observed between the samples (Fig. 8).

### Discussion

The accuracy of the flow microcalorimetric assay is high. The parameter most likely to cause any variation in accuracy would be a variation in antibiotic standards. The sensitivity of this assay is also very high. This can be seen from the results of the EM's of the bacteria where no visible damage to the bacteria can be detected within the sensitivity ranges of the assay implying that very small metabolic changes can be detected in the calorimeter. This is important as conventional microbiological assays can not compete with the sensitivity which is obtained at concentrations close to those found *"in vivo".*

Flow - microcalorimetry is a very simple method to use for routine assay of antibiotics, when all the conditions discussed previously are observed. For this assay the standard deviation was ± 2.9% which is a great improvement in reproducibilities from plate assays. (See table ref below).

| Criteria | Calorimetry | Plate Method |
|---|---|---|
| Inter Assay Reproducibiity % | 3 | 5-10 |
| Sensitivity u/ml | 0.1 | 20 |
| Range u/ml | 0.1-100 | 20-100 |
| Time per assay (h) | 1 | 1 6 |
| Capacity for automation | High | low |
| Convenience and simplicity | High | high |

### ASSAY OF MIXTURE OF ANTIBIOTICS

Even in the earliest days of the antibiotic era, despite dramatic cures of what had previously been fatal infections, it was apparent that in certain situations the use of a single drug led to unacceptable failure rates.

The exact role of antibiotic combinations in current therapy of clinical infections often remains controversial or unclear, in part because few tightly controlled studies have been carried out in this area.

Compatibility of different antiinfectives or indeed non compatibility involves several mechanisms, including sequential blockage in a given metabolic pathway e.g. trimethoprim and sulfonamides or changes in permeability to the bacterial surface by one drug e.g. penicillin and aminoglycosides or inhibition of inactivating enzymes e.g. penicillin and cloxacillin.

Results have varied when combinations of aminoglycosides and antipseudomonas penicillins have been used against Ps. aeruginosa and when combinations of cephalosporins and aminoglycosides have been used against Klebsiella species. However it is clearly unusual for non compatibility to be suggested by one technique when another method shows compatibility of an antibiotic combination against the same organism.

In the interpretation of the results of all tests of *in vitro* antimicrobial activity, attention must be paid to the overwhelming importance of host factors in the outcome of treatment of any infectious process. *In vitro* tests do not usually take into account the role of phagocytic cells, humoral antimicrobial substances, pharmacokinetic factors and pathopysiological conditions at the site of infections. There are techniques to establish bactericidal effects in blood but these have serious microbiological limitations as regards performance and interpretation of results. The results depend on how the test was done e.g. on the salt concentration and other environmental parameters. However some correlation could be achieved between the test and the clinical outcome.

No clinical studies have been carried out in which an antibiotic combination consistently compatible with another against an infecting organism was compared to a single agent with *in vivo* activity against the infecting pathogen.

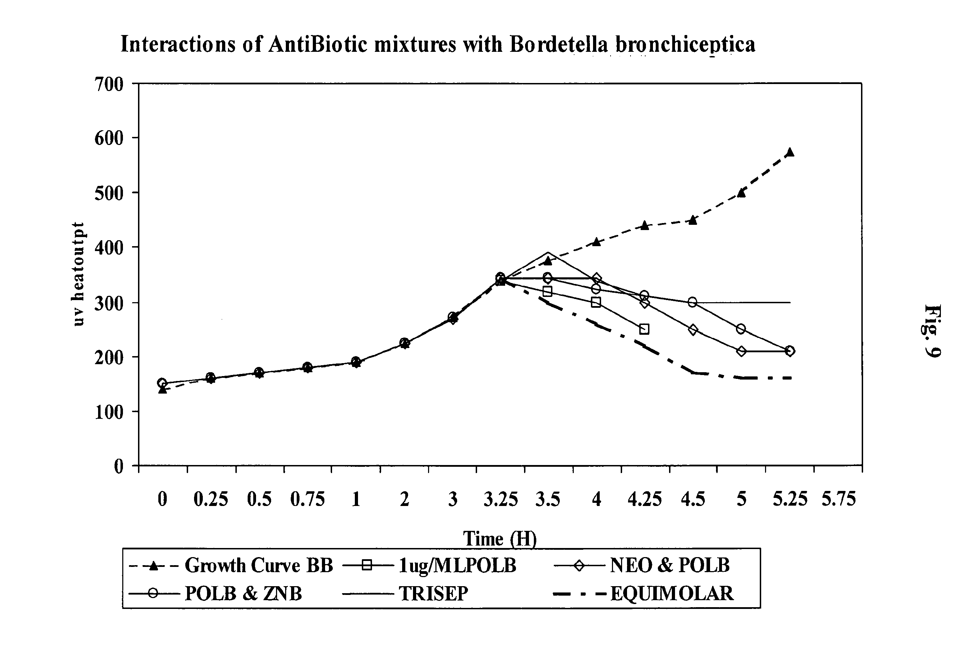

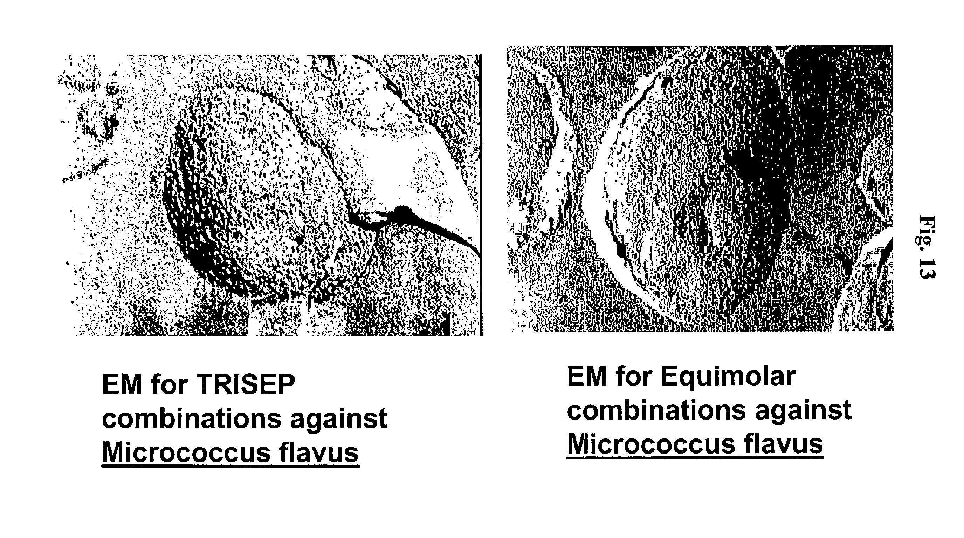

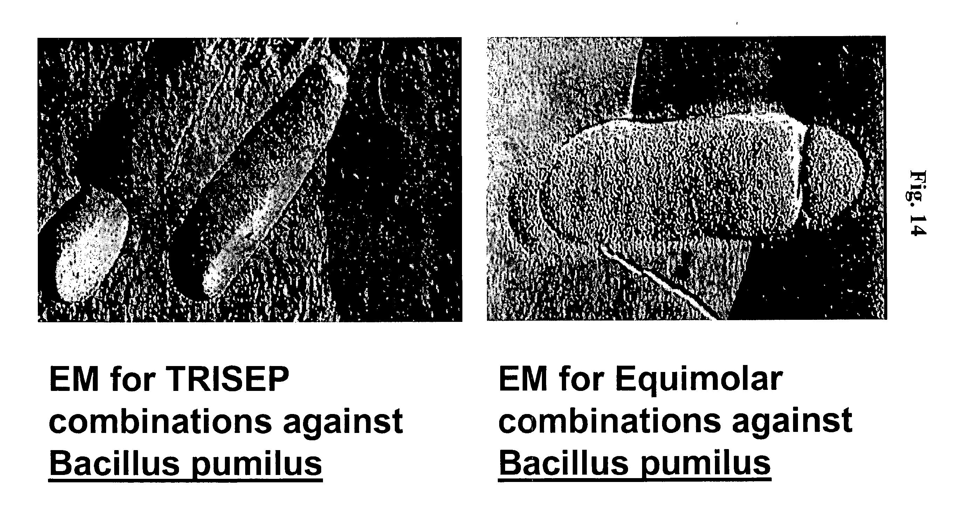

Biological compatibility and non compatibility of antibiotics can be demonstrated by micro calorimetry (see Figures 9 to 18). The antibiotics assayed in combination in this study were POLB, NEO and ZNB. These antibiotics were available as an antibiotic spray "TRISEP" from ICI and contained, on average, NEO 500.00 u, POLB: 150.000 u and ZNB 40000 u per can. Converted to mg the content was NEO 7.5 x 10² mg, POLB 1.7 x 10² mg and ZNB 6.32 x 10² mg. When assaying antibiotics in combination one antibiotic is usually added at some multiple of its MIC, whereas the amount of the second or third antibiotic added is often empirically gauged. A simpler and more regular picture of the interaction of mixtures of antibiotics would be achieved by using equimolar concentrations or concentrations taking into account the mode of action of the antibiotics. In this study antibiotic mixtures have been assayed in the proportions found in the Trisep mixture and in equimolar concentrations. The relative molecular masses (RMM) for the three antibiotics were as follows: ZNB: RMM 1477, POLB: RMM 1300 and NEO RMM 700.

### MIXTURES OF ANTIBIOTICS ASSAYED AGAINST BORDETELLA BRONCHISEPTICA (see Fig. 9)

| **Bacterial Concentration Bordetella Bronchiceptica** | **Antibiotic Concentration** | **Observed effect on p-t curves** | **Comments** |
|---|---|---|---|
| **As % of p-t curve** | **Ug/ml MIC NEO 0.25 ug/ml ZNB 100ug/ml POL 2ug/ml** | | |
| 50 | 0.25ug/ml Neo | No effect on p-t curve | |
| 50 | 100ug/ml ZNB | Slightly delayed peak | |
| 50 | Pol I ug/ml, Neo 4 ug/ml (Trisep conc) | Sharp decline in curve 10 min after addition of antibiotics response polB 0.7 ug/ml | Non compatibility of antibiotics demonstrated |
| 50 | POL 1 ug/ml ZNB 3.57ug/ml | 0.39 ug/ml POLB | Non compatibility of antibiotics demonstrated |
| 50 | All three antibiotics trisep combinations | 0.47 ug/ml Pol B | **2 hours after addition of antibiotic pt-power out put declined 10%, No return to baseline** |
| 50 | All three antibiotics equimolar amounts | 0.39 ug/ml PolB | **2 hours after addition of antibiotic pt-power out put declined 20% No return to baseline** |

### Fig 9

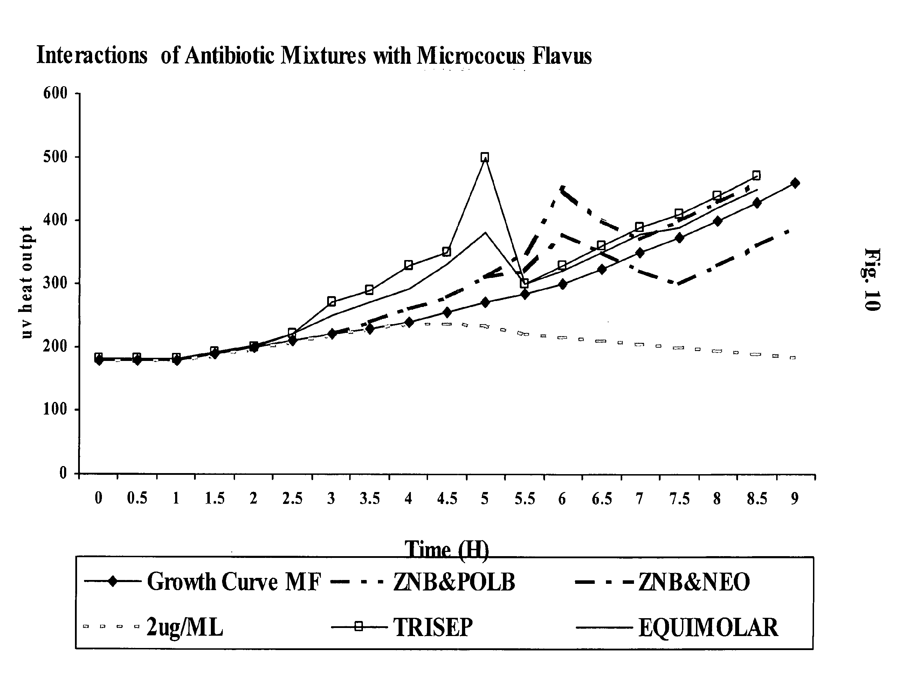

### MIXTURES OF ANTIBIOTICS ASSAYED AGAINST MICROCOCCUS FLAVUS (see Fig. 10)

| **Bacterial Concentration Micrococcus flavus** | **Antibiotic Concentration** | **Observed effect on p-t curves** | **Comments** |
|---|---|---|---|
| **As % of p-t curve** | **Ug/ml MIC NEO 4 ug/ml ZNB 0.2ug/ml POL 2ug/ml** | | |
| 0? | 4 ug/ml Neo | No effect on p-t curve | |
| 0? | 2ug/ml POLB | Slightly delayed peak | |
| 50 | 0.2 ug/ml ZNB 056 ug/ml POLB (trisep) | Delay in peak ZNB conc 0.11 ug/ml | Non compatibility of antibiotics demonstrated |
| 50 | NEO 0.226 ug/ml ZNB 0.2ug/ml (trisep) | 0.1 ug/ml ZNB | Non compatibility of antibiotics demonstrated |
| 50 | All three antibiotics trisep combinations | 1.3 times standard cuve | Growth promoting effect |
| 50 | All three antibiotics equimolar amounts | 0.1 ug/ml ZNB | **Small delay in peak compared to** standard curve |

### Fig 10

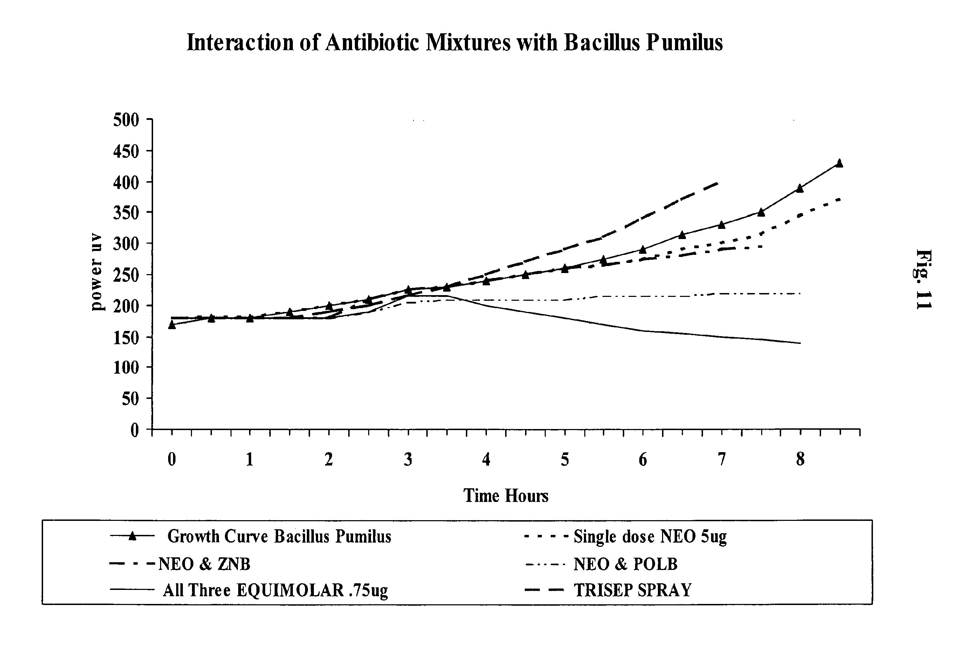

### MIXTURES OF ANTIBIOTICS ASSAYED AGAINST BACILLUS PUMILUS (see Fig. 11)

| **Bacterial Concentration Bacillus Pumilus** | **Antibiotic Concentration** | **Observed effect on p-t curves** | Comments |
|---|---|---|---|
| **As % of p-t curve** | **Ug/ml MIC NEO 1 ug/ml ZNB 20 ug/ml POL 20 ug/ml** | | |
| 0? | ZNB 20 ug/ml | No effect on p-t curve | |
| 0? | 20ug/ml POLB | Within 9 hours growth was inhibited completely | |
| 0 | 0.5 µg/ml) NEO + 0.125 µg/ml (0.006 MIC) POLB (TRISEP) | Compatibility Effect 1 ug/ml neomycin | compatibility of antibiotics demonstrated |
| 0 | And 0.25 MIC (0.5 µg/ml) NEO + 0.44 µg/ml 0.022 MIC ZNB (TRISEP). (trisep) | Compatibility Effect 1 ug/ml neomycin | compatibility of antibiotics demonstrated |
| 0 | All three antibiotics trisep combinations | 1.0 times standard cuve | **No effect** |
| 0 | All three antibiotics equimolar amounts neomycin 1 ug/ml, POLB,ZNB | 1.7ug/ml Neomycin | **Compatibility demonstrated** |

### Fig 11

### DISCUSSION

Compatibility was clearly demonstrated for all antibiotic combinations against B. pumilus except the Trisep mixture. These studies were all carried out at pH 8.00 as this was the most effective pH for neomycin. It seems likely that when using the Trisep mixture POLB and ZNB interfered with each other whereas an equimolar concentration no such effect was seen. The results suggest a critical range of concentration within which compatibility is observed.

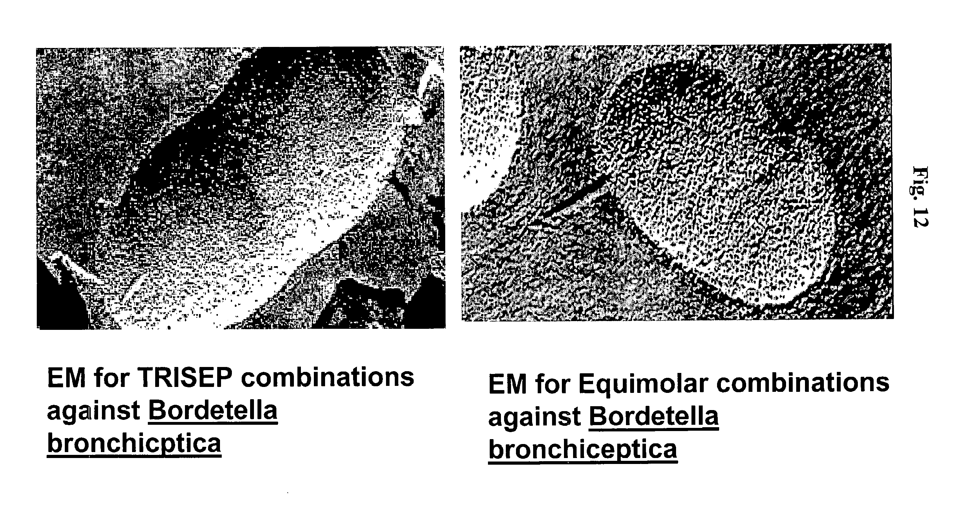

When assaying the three antibiotics against Bord. Bronch and M. flavus no such effect could be observed. In fact an indication of non compatibility could be observed for all these mixtures, and even a growth promoting effect at lower concentrations of antibiotics. It seems likely that the compatible action against Bacillus pumilus was due to POLB and ZNB facilitating the transport of neomycin into the cytoplasm by disrupting the bacterial membrane. All the above results have been verified by electron microscopy (see Figs. 12 to 14). It has been shown by EM that ZNB had some effect on E. coli as protrusions have been observed on the bacterial membrane after treatment with ZNB.

A possible assay of POLB and ZNB in the combinations used here could probably be achieved using Bord. bronch and M. flavus respectively as test organism. The assays would not be as sensitive as the assays for the individual antibiotics but a linear relationship between dose and response may be achievable. For the neomycin assay, using B. pumilus as test organism, a great variation in concentration of the antibiotics would have to be carried out to see at which concentrations, if any, an assay of all three antibiotics would be possible i.e. that an assay could be developed for neomycin with a constant response for ZNB and POLB.

### RESPIRATION AND GROWTH OF E.COLI

E.coli was suspended in 10% DMSO prior to freezing and the viable counts were for batch 1: pre-freeze: 4.0 ± 0.6_ x 10¹⁰ cfu/ml and post -freeze 2.0 ± 0.44 x 10¹⁰ cfu/ml and for batch 2: pre-freeze: 3.45 ± 0.58 x 10¹⁰ cfu/ml and post-freeze 2.93 ± 0.43 x 10¹⁰ cfu/ml. The freezing rate for both batches was 20°C/min the survival rate was 50% for batch 1 and 85% for batch 2. These differences may have been due to batch 1 being kept for 24h at 4°C before being frozen.

The respiration experiments were carried out in glucose buffer at different pH. The respiration curves obtained from batch 1 (inoculum 2 x 10¹⁰ cfu/ml) and batch 2 Inoculum 3.2 x 10¹⁰ cfu/ml) were essentially similar and a mean was used as a reference). The respiration experiments were carried out at pH 7 for polymyxin and Znbacitracin and at pH 8 for neomycin. No differences in p-t control curves could be observed at pH 7 or pH 8.

The p-t curves rose and reached a plateau as has been observed for respiration of yeasts and some 20 min later fell to a lower plateau value. Prior incubation of the cells in the buffered glucose for periods up to 20 min before presentation to the calorimeter could reduce or entirely remove this effect.

### INTERACTION OF POLYMYXIN B, NEOMYCIN AND ZNBACITRACIN WITH RESPIRING

### E. COLI

| **Bacterial Concentration E. coli** | **Antibiotic Concentration** | **Observed effect on p-t curves** | **Comments** |
|---|---|---|---|
| **As % of p-t curve** | **Ug/ml MIC&MBC NEO 5900 ug/ml ZNB > 1000 ug/ml POL 6.3ug/ml** | | |
| respiration | **POLB 6.3 ug/ml** | Reduced height of p-t curve initially and returned to baseline within 1 hour | **Bacteriocidal action demonstrated** |
| respiration | NEO 500 ug/ml | Increase in heat output Linear dose-response between 10-100ug/ml | **Consistent with increased rate of RNA synthesis** |
| respiration | ZNB 300 ug/ml - 1000 ug/ml | No response | Resistance demonstrated |
| respiration | : POLB: 20µg/ml NEO: 80.8µg/ml and ZNB 71.4µg/ml (TRISEP combination). | POLB - NEO longer plateau drop after 20 min to baseline POLB ZNB NEO ZNB NEO response curve | POLB/NEO interaction: delayed response compared to polymyxin alone but drop to baseline no increase in power NEO/ZNB no interference of ZNB with NEO action higher output but not as high as with NEO alone |
| respiration | All three antibiotics trisep combinations | Polymyxin curve with delayed return to baseline 20 min | **Effect not as good as polymxin alone or Neomycin alone** |
| respiration | All three antibiotics equimolar amounts | Polymyxin curve with delayed return to baseline 9 min | **Effect not as good as polymxin alone or Neomycin alone** |

### Fig 15, 16 and 18

### Interaction of Polymyxin B with E.coli

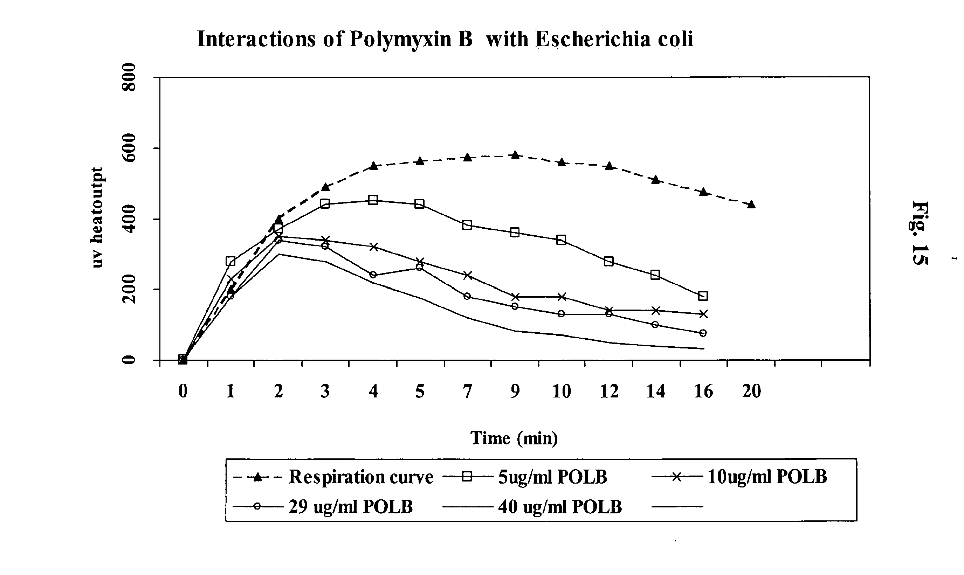

The MIC and MBC for E.coli was found to be 6.3µg/ml. Polymyxin B reduced the initial peak height and thereafter the p-t curve returned to baseline (Fig. 15). This effect was in accordance with the mode of action of polymyxin as the bacteriocidal action was very rapid. This was also observed when using Bord. Bronch as test organism.

### Interaction of Neomycin with E.coli

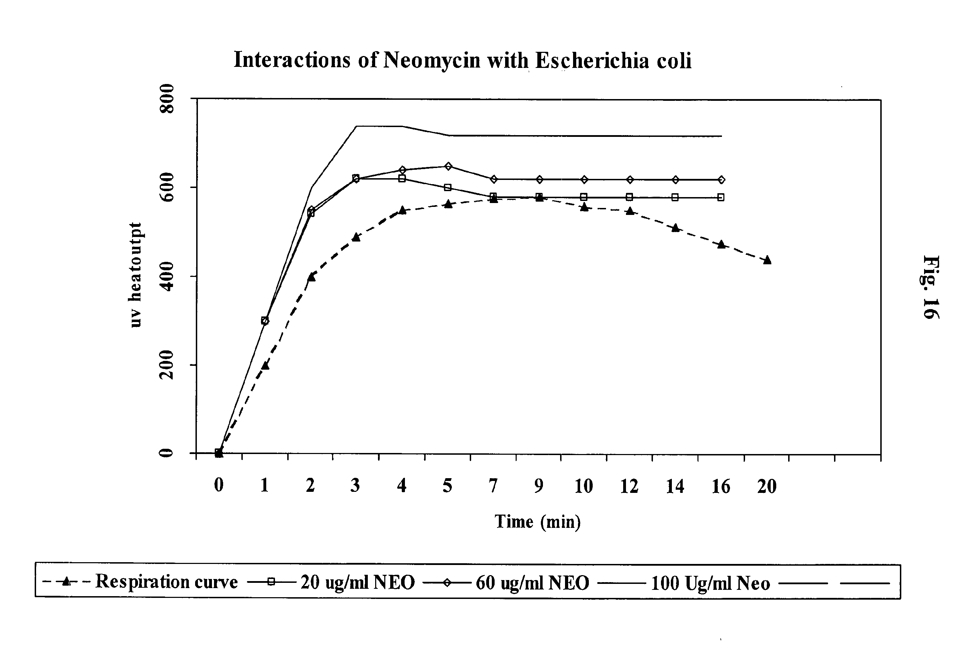

The effect of neomycin on E.coli is given in Fig. 16. In contrast to polymyxin, neomycin caused an increase in power which confirms that Neomycin fundamentally stimulates RNA synthesis and only inhibits it when present in very large amounts when added to respiring E.coli. (MIC: 500µg/ml). A linear relationship between dose and the reciprocal of increase in response from the control curve was found between 20-100µg/ml neomycin.

### Interaction of Znbacitracin with E.coli

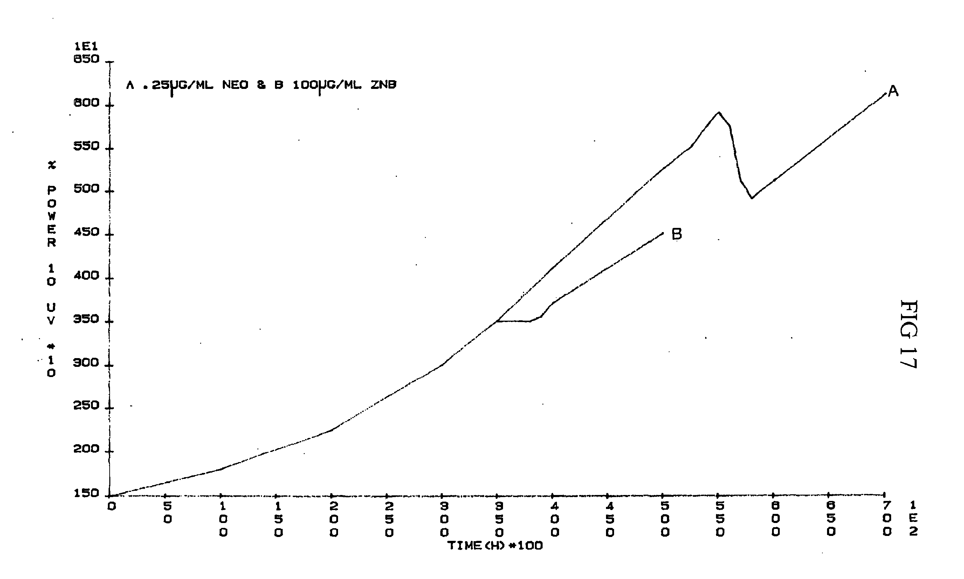

No response could be observed on addition of Znbacitracin to respiring E.coli. The highest concentration added was 300µg/ml (MIC> 1000µg/ml). ZNB has been reported to be inactive against E.coli. However electron micrographs have shown that ZNB had an observable effect on E.coli i.e. did actually cause protrusions to occur on the bacterial membrane. This response was not lethal for the bacterium but small changes in heat could possibly have been expected to occur. This has in fact been observed when ZNB was added to growing cultures of Bord. bronch. a small plateau was reached before growth reoccurred (see Fig. 17).

### Interaction of Mixtures of Antibiotics

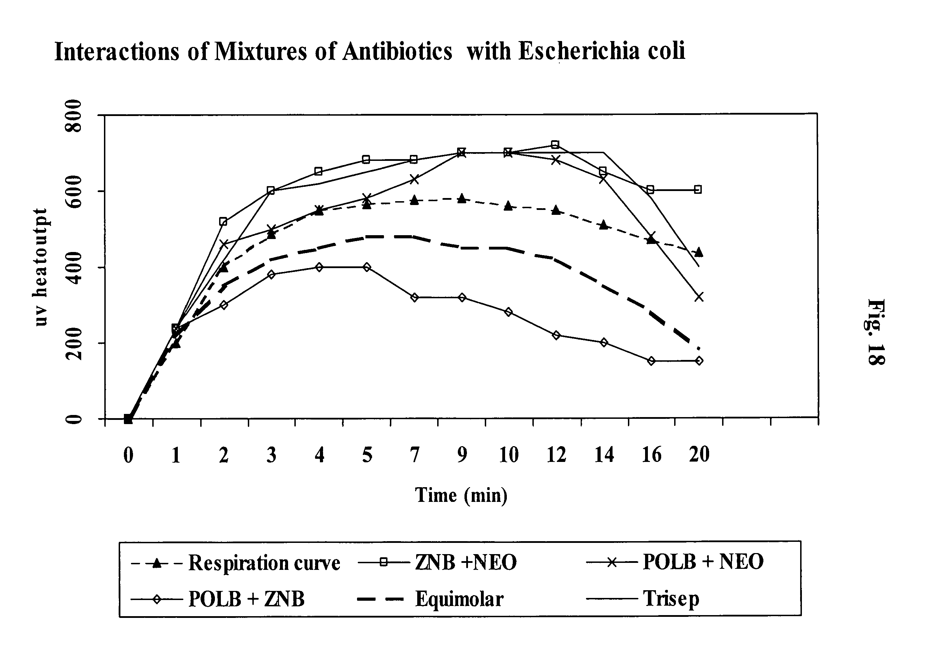

Fig. 18 shows the p-t curves for the effect of mixtures of antibiotics on E.coli. The concentrations were: POLB: 20µg/ml NEO: 80.8µg/ml and ZNB 71.4µg/ml (TRISEP combination). Fig. 18 shows the p-t curve for ZNB + NEO; when compared with the p-t curves for neomycin alone it can be seen that this p-t curve was essentially similar to that for neomycin alone (20µg/ml NEO). Fig. 18 shows the effect of POLB + NEO on E.coli. When comparing the effect of the mixture to that of POLB alone (Fig. 15) it can be seen that the effect of the two antibiotics was not comparable to the polymyxin interactions as the kinetics of the interactions seemed to have changed resulting in a plateau value for much longer periods than found for the polymyxin alone followed by a decline to a baseline value after 20 min. When compared to the neomycin interactions (Fig. 16) the p-t curve for NEO and POLB was very different.

Fig. 18 shows the effect of polymyxin and Znbacitracin in combination and when compared with that for polymyxin alone (Fig. 9) the p-t curve is seen to be similar in shape to that for polymyxin but shows a higher output. This response was equivalent to a response of 15µg/ml polymyxin and suggests non compatibility.

Fig. 18 shows the effects of all three antibiotics added to respiring E.coli (A) as the TRISEP mixture and (B) as the equimolar mixture. The TRISEP mixture shows a response similar to that of POLB + NEO apart from showing a decline to baseline which was at a slower rate whereas the equimolar mixture at (20µg/ml POLB) showed a polymyxin type curve with a plateau value very similar to that of the control curve, but then, after 9 min, a steady decline to baseline.

The heat effects observed with polymyxin B were those that would be expected of membrane active antibiotics; i.e. severe reduction in power output followed by a return to baseline signifying death or lysis of cells after a breakdown in membrane potential and leakage of cell constituents from the cytoplasm of the cell. The polymyxin type interactions showed kinetics which were essentially simply and which have been observed with antifungal antibiotics. Neomycin, in contrast, caused an increase in power output also observed in growth studies with Basillus Pumilus which would indicate an immediate effect on RNA synthesis and a very good reason not to use this antibiotic

That ZNB had no effect on power output was not surprising since the antibiotic has been reported to be active only against Gram positive organisms. The assays for POLB were not particularly sensitive (3µg/ml) but had the advantage of being rapid. They do compare favourably with the assay using Bord. Bronch. as test organism for this reason. Which assay to use would depend entirely on the sensitivity required. The reproducibilities for the E.coli assay were also smaller than those for the Bord. bronch. assay. The neomycin assay was much more sensitive when using B.pumilus as test organism. However the assay with E.coli was possible at concentrations much lower than the measured MIC (500µg/ml). It can be concluded that these two assays would be suitable for screening antibiotics where sensitivity is not essential, since they are very rapid (1h).

It can be concluded from Fig. 18 that all the antibiotics assayed together show (two by two) possible antagonistic effects. The effect observed with neomycin and polymyxin was an initial increase in peak deflection followed by a gradual decrease to baseline. This suggested that the initial effect on E.coli was due to neomycin followed by the effect of polymyxinFor POLB + NEO assayed against E.coli it can be observed that the rate of decline of power was identical to that noted for the antibiotic on its own suggesting that these interactions followed the same order of kinetics at this point. That polymyxin and bacitracin assayed together showed non compatibility was not surprising since a possible explanation could be that competition for binding sites on the bacterial membrane would occur. This effect was also in agreement with the effect observed for this mixture when assayed against Bord. bronch. and M. flavus. This possible competitive effect could result in the inhibition of binding of the most active agent. When all three antibiotics were added together either as the TRISEP combination or as the equimolar combination, p-t curves resulted that were different from those observed either for the antibiotics added singly or in binary combinations.

These results form an excellent basis for rapid compatibility testing of anti-infective agents both during development and also as part of clinical practice in hospitals, doctors offices, dental clinics, veterinary clinics and in developing countries.

A real life example of issues relating to antibiotic treatment and their adverse effects that causes death (in an AIDS-like fashion, but would indicate stimulation of both prokaryotic and eukaryotic mRNA synthesis and consistency with what was observed during early Aids cases) is shown by the following case study.

### An Animal case study

A domestic cat was given a mixture of antibiotics as eye drops in response to a scratch to the eye. The cat had a major adverse reaction which caused serious weight loss (that later caused its death). The cat was given 30 days of vancomycin treatment without any effect to the eye. Previously the cat had never been given milk (as many animals are allergic to cow's milk). The cat was now given full cream milk as part of a regimen to promote weight gain (the cat also continued to eat its usual amount of daily cat food throughout the period of weight loss). Initially weight was gained but within 24 hours weight loss resumed (presumably because the pathogenic bacteria/viruses had found a way of utilising lactose). An autopsy indicated bone-cancer as cause of death. The body wasting caused by the weight loss was very similar to that observed in early AIDS cases. However the cat had been regularly vaccinated against HTLV 1 through its 16 year life (and had never been ill in that period).

## Claims

1. Method of diagnosing the presence of, or susceptibility to, a condition caused by a pathogenic microorganism in an individual, which method comprises determining whether or not a sample from the individual comprises said microorganism, wherein said determining is performed by placing the sample in conditions in which any said microorganism present in the sample is able to grow, and determining the presence of any said microorganism by detection of the heat generated by said growth using a calorimeter.

2. Method according to claim 1 which further comprises analysis of the heat output data to:
- determine the quantity of microorganism in the sample and thus to deduce whether or not the microorganism is present in sufficient quantity to cause a disease, and/or
- to determine the species of microorganisms in the sample, and/or
- to determine from the number of different species of microorganism in the sample.

3. Method according to claim 1 or 2 wherein the sample is a swab or is a blood, plasma, cell (infected or uninfected), urine, stool or saliva sample.

4. Method according to any one of the preceding claims wherein the sample is placed in said conditions for 1 to 24, such as 4 to 12, hours.

5. Method according to claim any one of preceding claims further comprising selecting an anti-microorganism compound (optionally an antibiotic) or a combination of anti-microorganism compounds to administer to the individual based on the results of said analysis.

6. Method of determining whether an anti-microorganism or anti-infective compound is able to cause growth of the microorganism/virus/mammalian cell comprising adding the compound to the microorganism, optionally at a pre-determined low concentration threshold, such as < 10xMIC, <8xMIC, < 2xMIC or < 1xMIC, to determine whether or not growth or heat output of the microorganism/virus/mammalian cell occurs in response to the addition of the compound, wherein growth of the microorganism is optionally measured by detecting heat output from the microorganism/virus/mammalian cell using a calorimeter, and wherein the compound may be alone or in a combination with 1, 2 or more other anti-microorganism or anti-infective agents.

7. Method according to claim 6 wherein if the said compound is found to cause increased growth of microorganisms at the pre-determined low concentration, then administering the compound to an individual suffering from a condition caused by the microorganism or at risk of such a condition, at a high dosage, but optionally not at dosages causing increased respiration of the said compound.

8. Method of determining a suitable administration regimen for a patient comprising taking a sample from a patient on a first administration regimen and detecting the effectiveness of the first administration regimen by measurement of the immune response, microorganism load or numbers of malignant cells in a sample from a patient on the first administration regimen, wherein measurement is performed by using calorimetry, and then optionally if the first administration regimen is found to be of low effectiveness selecting a second administration regimen for the patient.

9. Method according to claim 8 wherein the first and second administration regimen differ in respect to the drug or combination of drugs which are administered.

10. Method according to claim 8 or 9 wherein the patient is infected with HIV.

11. Method of diagnosing cancer in an individual, which method comprises determining whether or not a sample from the individual comprises malignant cells, wherein said determining is performed by placing the sample in conditions in which any said malignant cells present in the sample are able to grow, and determining the presence of any said malignant cells by detection of the heat generated by said growth using a calorimeter.

12. Method of determining possible deleterious effects of an anti-infective compound on a eukaryote comprising determining whether said compound:
- causes increased growth of the eukaryote or cells from the eukaryote,
- causes cells of the eukaryote to become cancerous, or
- causes increased production of RNA or DNA or protein in cells of the eukaryote,
wherein said determining is optionally performed using a calorimeter.

13. Method according to claim 12 further comprising elucidating the mechanism of increased growth, optionally by measuring the RNA or DNA synthesis and/or protein synthesis levels of the microorganism or eukaryote.

14. Method of selecting an anti-microorganism or anti-infective compound to administer to an individual suffering from a condition caused by the microorganism or at risk of such a condition, comprising testing whether a candidate anti-microorganism or anti-infective compound is able to cause growth of the microorganism by the method of claim 12, and selecting a compound which:
- does not cause growth or causes a low level of growth, or
- only causes growth below a pre-determined low concentration of compound,
wherein optionally the compound is then administered to the individual.

15. Method of administering an anti-microorganism or anti-infective compound to an individual suffering from, or at risk from, a disease condition according to an improved schedule which reduces detrimental effects, wherein in the improved schedule one or more dosages are increased by at least 1.5 fold in comparison to an existing schedule.

16. Method according to claim 15 wherein the anti-microorganism compound is one which acts at the ribosome of the microorganism.

17. Method according to claim 15 or 16 wherein at least 2 or more dosages are increased by at least 2 fold in the schedule.

18. Method according to any one of claims 15 to 17 wherein the said compound is initially administered in accordance with the existing schedule, and is then subsequently administered to the same individual in accordance with the improved schedule.

19. Method according to any one of claims 15 to 18 wherein the improved schedule comprises 2, 3, 4, 5 or more administrations and/or at least 1, 2, 3, 4, 5 or more other anti-microorganism agents are also administered to the individual during therapy.

20. Method according to any one of claims 15 to 19 wherein mixtures of compounds are administered to the individual (optionally including compounds for treatments of different conditions).

21. Method of measuring compatibility between two or more different compounds comprising providing the compounds to a cell line, cancer cell, microorganism cell or virus sample in vitro and detecting said compatibility by detection of the heat generated in the sample when the compounds act on said cell, wherein said heat is detected using a calorimeter, and wherein each compound has an anti-cancer, anti-microorganism or anti-viral activity respectively, to thereby determine whether the compounds will be compatible with each other for use in vivo.

22. Method according to claim 21 wherein non-compatibility of said compounds is investigated by determining whether the activity of the compounds is reduced when they are used in combination with another said compound.

23. Method of quantitatively determining
- the resistance of a cell line, cancer cell or microorganism sample to an anti-cancer or anti-microorganism compound(s), or
- the effect on the growth of a cell line, cancer cell or microorganism sample of an anti-cancer or anti-microorganism compound(s),
comprising detecting the heat generated in the sample when the compound(s) acts on the sample, to thereby determine the resistance or effect on growth, wherein the heat is detected using a calorimeter.

24. Method according to any one of claims 21 to 23 comprising carrying out detections on at least three said samples wherein at least two of the compounds are present at different ratios in the samples.

25. Method according to any one of claims 21 to 24 wherein the microorganism is a pathogenic microorganism and/or is a bacterium, fungus or virus and/or is a drug resistant strain.

26. Method according to any one of claims 21 to 25 wherein the microorganism is vancomycin resistant enterococci, pencillin resistant Streptococcus pneumoniae, methicillin resistant Staphylococcus aureus, multi-drug resistant gram-negative bacillus, multi-drug resistant Mycobacterium tuberculosis, multi-drug resistant gram-positive bacteria, Achromobacter, acinetobacter, aerococcus, aeromonas, alicalignes, bacillus, bacteroides, burkholderia, chryseobacterium, citrobacter, clostridium, corynebacterium, enterobacter, enterococcus, escerichia, fusobacterium, haemophilus, hafnia, klebsiella, listeria, micrococcus, moraxella, morganella, pantoea, peptococcus, peptostreptococcus, proteus, providencia, pseudomonas, salmonella, serratia, staphylloccocus,stenotrophomonas, streptococcus multi-drug resistant malaria parasite or HIV, HSV, RSV or viruses which are members of the families Picornaviridae (e.g. polioviruses or rhinoviruses); Caliciviridae; Togaviridae (e.g. rubella virus or dengue virus); Flaviviridae; Coronaviridae; Reoviridae (e.g. rotavirus.); Birnaviridae; Rhabodoviridae (e.g. rabies virus); Orthomyxoviridae (e.g. influenza virus types A, B and C); Filoviridae; Paramyxoviridae (e.g. mumps virus, measles virus, respiratory syncytial virus or parainfluenza virus); Bunyaviridae; Arenaviridae; Retroviradae (e.g. HTLV-I ; or HTLV-II); Papillomavirus, or a tick-boume encephalitis virus.

27. Method according to any one of the claims 21 to 26 wherein the method is carried out to determine the most effective ratio in which to administer the compounds to an individual, and optionally administering the compounds in said ratio.

28. Method according to claim 27 which further comprises dispensing the compounds into a composition in the ratio determined to be most effective.

29. Method of administering an anti-microorganism or anti-infective compound to a neonate or child individual suffering from, or at risk from, a disease condition according to an improved schedule which reduces detrimental effects, wherein in the improved schedule one or more dosages are decreased by at least 1.5 fold in comparison to an existing schedule, wherein the individual is optionally below the age of 10 or below the age of 5 or 1 years old.

30. Method according to any one of the preceding claims wherein the individual, patient or eukaryote is a human, animal, mammal, primate, bird, domestic pet, agricultural animal, wild animal, sporting animal or fish or where the sample is from an individual of any such organism.

31. Method according to any one of the preceding claims wherein the individual, patient or eukaryote is of any of the following species, or where the sample is from an individual of any of the following species: horse, pig, cow, dog, cat, sheep, goat, turkey, chicken, camel, llama, deer, duck, fish, monkey, mouse, rat, guinea pig or hamster.

32. Method according to any one of the preceding claims wherein the calorimeter is able to detect changes in heat flow of 25 nanocalories/sec and/or heat effects of 10 microcalories.

33. Method according to any of the preceding claims which is used in diagnosis, therapy or clinical investigation, optionally in a hospitals, GP's office or dentist surgery.

34. Method according to any one of claims 1 to 32 which is used in diagnosis, therapy or clinical investigation in a veterinary office or farm.

35. Kit for carrying out a method according to any of the preceding claims comprising a calorimeter suitable for use in said method.
